(19)

Europäisches
Patentamt
European
Patent Office
Office européen
des brevets

(11) **EP 2 544 003 A1**

(12) **EUROPEAN PATENT APPLICATION**

(43) Date of publication:
**09.01.2013 Bulletin 2013/02**

(51) Int Cl.:
*G01N 33/574* (2006.01)    *C12N 15/113* (2010.01)

(21) Application number: **11382228.2**

(22) Date of filing: **07.07.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **CNIC, Fundacion Centro Nacional de
Investigaciones
Cardiovasculares Carlos III
28029 Madrid (ES)**

(72) Inventors:
• **Del Pozo Barriuso, Miguel Ángel
28029 Madrid (ES)**
• **Goetz, Jacky Gerard Fernand
28029 Madrid (ES)**

(74) Representative: **Pons Ariño, Angel
Pons Patentes y Marcas Internacional, S.L.
Glorieta Rubén Dario 4
28010 Madrid (ES)**

(54) **Caveolin-1 in tumor-associated fibroblasts as biomarker for tumor progression**

(57)    The present invention refers to the use of caveolin-1 in tumor-associated fibroblasts as a biomarker of tumor progression, preferably using smooth muscle actin as biomarker for tumor-associated fibroblast. The present invention also relates to a method for the diagnosis and/or prognosis of tumor progression comprising the detection and/or quantification of caveolin-1 expression in the tumor-associated fibroblasts of an isolated sample. The present invention also refers Caveolin-1 inhibitors for use as a medicament and in the prevention of tumor progression.

EP 2 544 003 A1

**Description**

**FIELD OF THE INVENTION**

The present invention relates to the use of caveolin-1 as a biomarker for tumor progression when expressed in tumor-associated fibroblasts. The present invention also relates to a method for the diagnosis and/or prognosis of tumor progression comprising the detection and/or quantification of caveolin-1 expression in the tumor-associated fibroblasts of an isolated sample.

**BACKROUND OF THE INVENTION**

*In vivo*, cells interact with a three-dimensional (3D) microenvironment (Yamada and Cukierman, 2007 Cell 130, 601-610). The mechanical force of these interactions, by altering tissue tension, acts as a molecular switch that determines cell fate (Engler et al., 2009 Science 324, 208-212). Tension generated in an extracellular microenvironment induces and cooperates with opposing forces applied by cells (mechanoreciprocity). In embryogenesis such tensile forces govern tissue organization (Krieg et al., 2008 Nat. Cell Biol. 10, 429-436), and mammary acinar architecture relies on matrix compliance and cell tension (Ronnov-Jessen and Bissell, 2008 Trends Mol. Med. 15, 5-13).
Microenvironment-mediated tensile forces also contribute to disease. Matrix stiffness promotes breast cancer progression via mechanoreciprocal induction of Rho-dependent cell contractility (Levental et al., 2009 Cell 139, 891-906). The microenvironment is also important for tumor invasion and metastasis: tumor cells (TCs) migrate along tracks made of extracellular matrix (ECM) collagen fibers (Friedl and Gilmour, 2009 Nat. Rev. Mol. Cell Biol. 10, 445-457), and whereas reticular collagen surrounding mammary glands restrains invasion, Rho mediated alignment of dense collagen fibers perpendicular to the tumor boundary promotes it (Provenzano et al., 2008 Biophys. J. 95, 5374-5384). Activated fibroblasts facilitate tumor cell invasion through protease- and force-dependent generation of ECM tracks (Gaggioli et al., 2007 Nat. Cell Biol. 9, 1392-1400). Carcinoma-associated fibroblasts (CAFs), also called tumor-associated fibroblasts (TAFs) and mesenchymal stem cells, via paracrine cytokine signaling, promote tumor growth, invasion, and metastasis (Karnoub et al., 2007 Nature 449, 557-563; Orimo et al., 2005 Cell 121, 335-348).
Caveolin-1 (Cav1), the major component of endocytic caveolae plasma membrane (PM) invaginations, has many functions outside caveolae (Parton and Simons, 2007 Nat. Rev. Mol. Cell Biol. 8, 185-194). Cav1 activates Rho by regulating its endogenous inhibitor p190RhoGAP (p190) and assists in focal adhesion (FA) stabilization required for directional cell migration (Goetz et al., 2008 J. Cell Biol. 180, 1261- 1275; Grande-Garcia et al., 2007 J. Cell Biol. 177, 683-694). The role of Cav1 in tumor progression remains unclear. In most primary tumors Cav1 levels decrease, allowing proliferation, anchorage independence, and angiogenesis, whereas metastasis correlates with Cav1 re-expression, promoting invasion, survival, and multidrug resistance (Goetz et al., 2008 Cancer Metastasis Rev. 27, 715-735). Most studies have focused on Cav1 expression in TCs, with no attention paid to a possible role in the tumor microenvironment.

**SUMMARY OF THE INVENTION**

The present invention provides a useful and reliable biomarker of tumor progression. The inventors have found that the higher expression of caveolin-1 (Cav1) in tumor-associated fibroblasts (TAFs) correlates with a poorer prognosis, increased tumor progression, higher invasive and metastatic tumor capacity and decreased patient survival. Also, the inventors have found that the inhibition or absence of Cav1 in the tumor microenvironment favours tumor encapsulation, and therefore prevents tumor progression and metastasis.
A first aspect of the present invention relates to the use of Cav1 in TAFs as a tumor progression biomarker. Preferably, at least one further known biomarker for TAFs selected from smooth muscle actin (SMA), CD90 and vimentin is used. More preferably, the TAFs biomarker SMA is further used in order to specifically localize Cav1 expression in TAFs.
A second aspect of the present invention relates to a method for the diagnosis and/or the prognosis of tumor progression, comprising the following steps: (a) detection and/or quantification of an expression product of *Cav1* gene in tumor-associated fibroblasts of a sample isolated from a subject; (b) comparison of the expression levels of Cav1 detected and/or quantified in step (a) with standard values; (c) finding a significant overexpression of Cav1 in the comparison of step (b); and (d) attributing the significant overexpression of Cav1 found in step (c) to a poor prognosis.
A third aspect of the present invention relates to a kit consisting essentially of specific primers and/or probes and/or antibodies for detecting and/or quantifying *caveolin-1* expression; and specific primers and/or probes and/or antibodies for detecting and/or quantifying the expression of at least one of the following genes: SMA, CD90 and vimentin, preferably of SMA.
A fourth aspect of the present invention relates to Cav1 inhibitors for use as a medicament. A fifth aspect of the present invention relates to Cav1 inhibitors for use in the prevention of tumor progression.
A sixth aspect of the present invention relates to p190RhoGAP-A (hereafter called p190) agonists for use as a medicament. A seventh aspect of the present invention relates to p190 agonist for use in the prevention of tumor progression.

An eighth aspect of the present invention relates to Rho-A inhibitors for use in the prevention of tumor progression.

**BRIEF DESCRIPTION OF THE DRAWINGS**

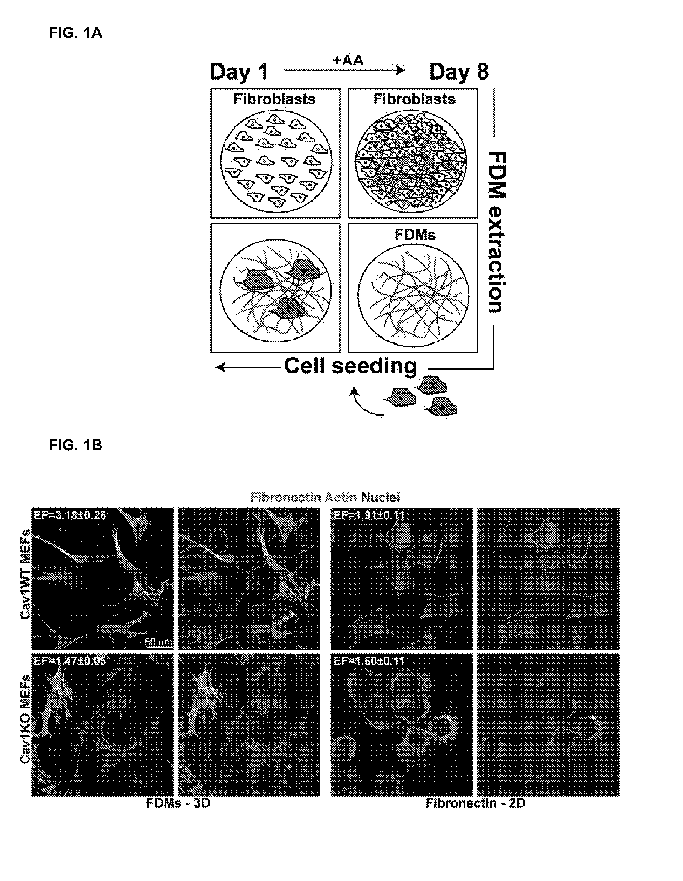

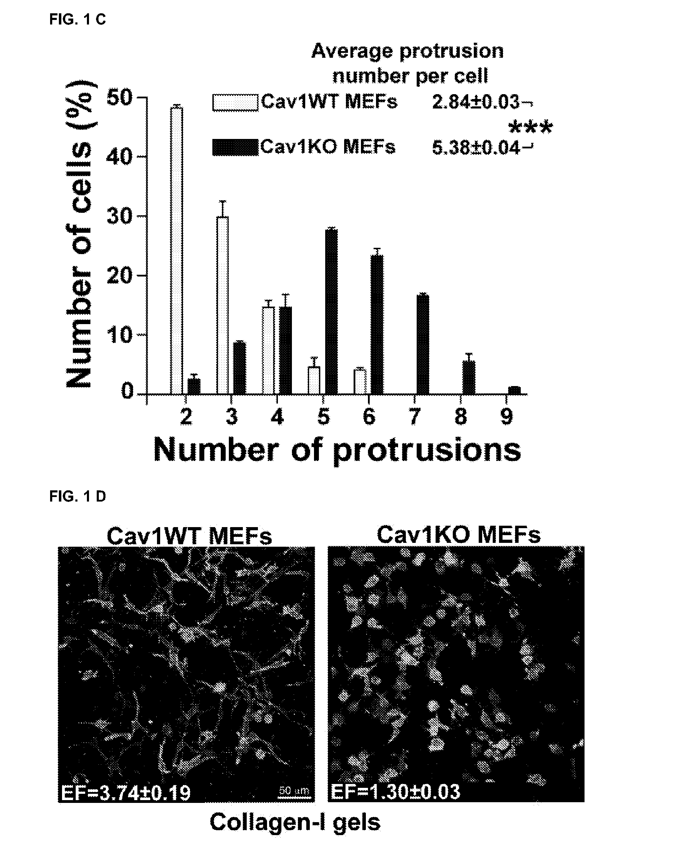

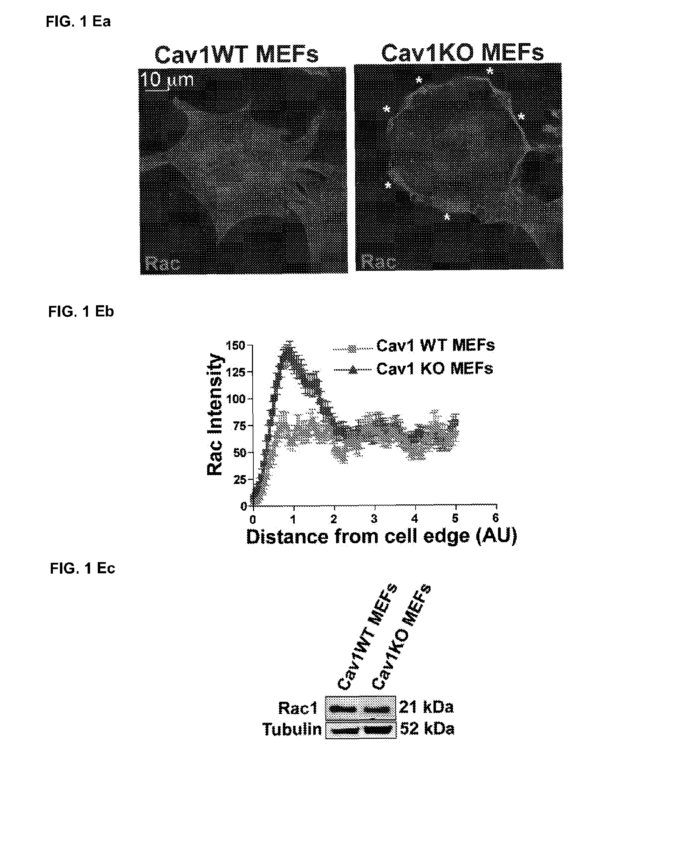

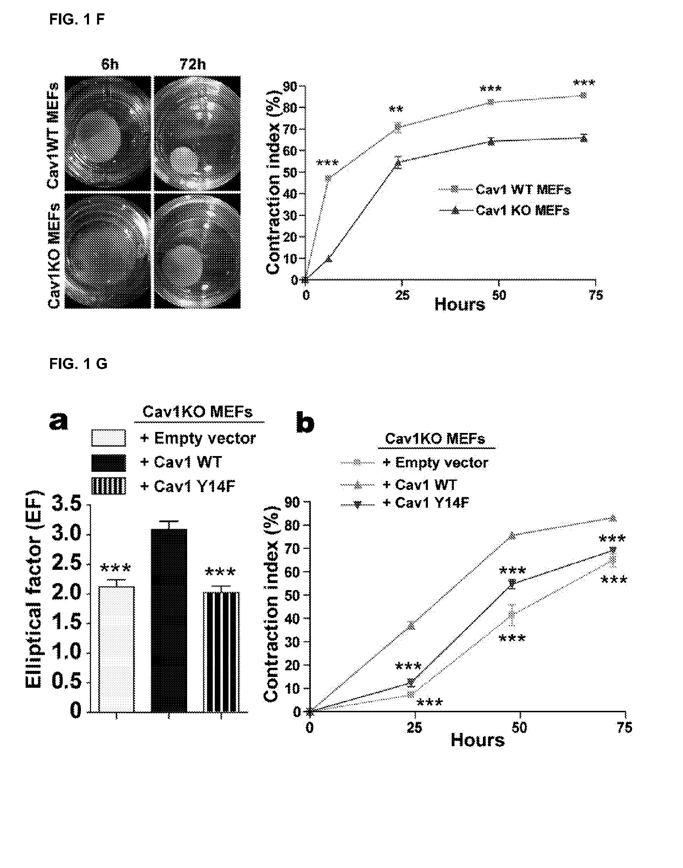

**Figure 1.** Cav1-Regulated Contractility Controls Matrix-Induced Cell Morphology and Reciprocal Interaction with the 3D Microenvironment (A) Fibroblast-Derived Matrices (FDMs) were generated from NIH 3T3 cells cultured with daily ascorbic acid (AA) supplement. (B) Cav1WT (Cav1 Wild Type) and KO (Knock Out) MEFs (Mouse Embyonic Fibroblasts) were plated (4 hr) on NIH 3T3 FDMs (3D) or FN (Fibronectin) (5 mg/ml, 2D) and labeled as indicated. Elliptical factors (EF) were calculated. (C) Quantification of protrusions per cell (means indicated). Representative Cav1WT and Cav1KO cells (asterisks mark protrusions) are shown. (D) Cav1WT and KO MEFs were embedded (6 hr) in Col-I (Collagen type I) gels (1 mg/ml). EFs (Embryonic Fibroblasts) are indicated. (E) Rac1 distribution in Cav1WT and KO MEFs. (a) Rac1 immunostaining in cells plated (4 hr) on FN. Asterisks mark Rac1 foci. (b) Rac1 pixel intensity fromthe cell edge to the nucleus. (c) Immunoblot showing total Rac1 expression. (F) Gel contraction by Cav1WT and KO MEFs embedded in Col-I gels. (G) Cav1-dependent cell elongation (a) and gel contraction (b) require Cav1-Tyr14. Data are represented as mean $\pm$ standard error of the mean (SEM).

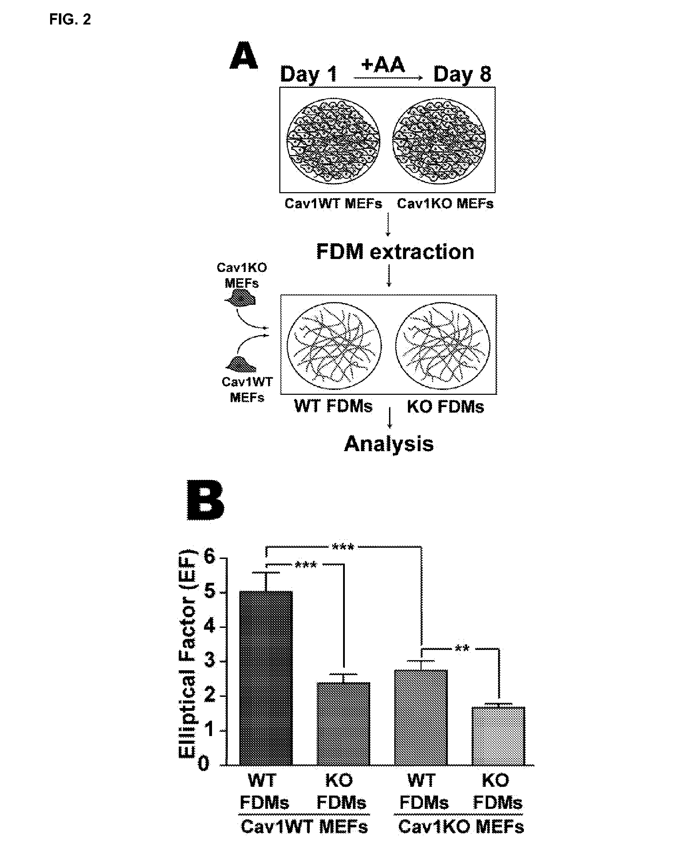

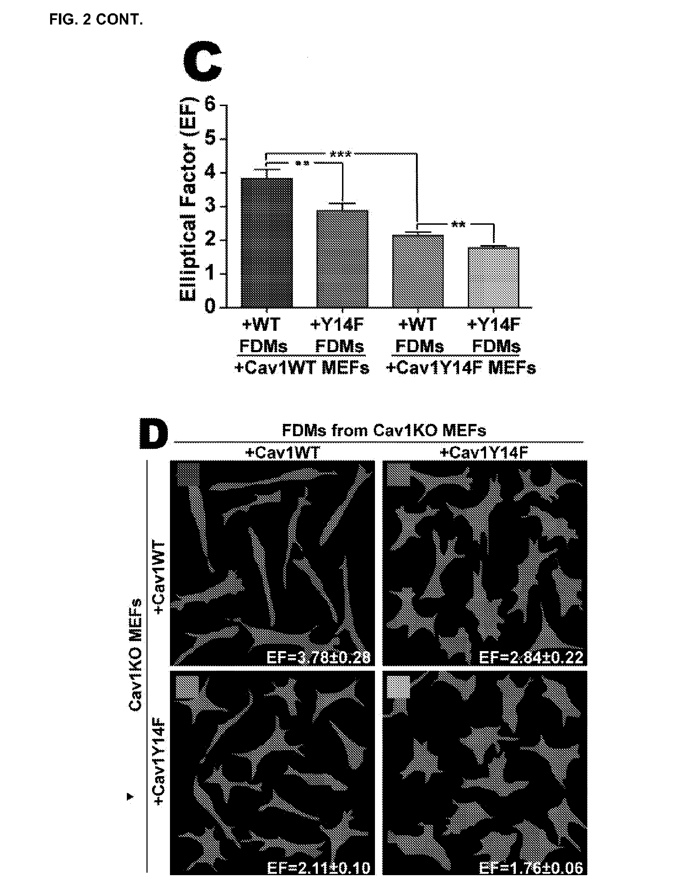

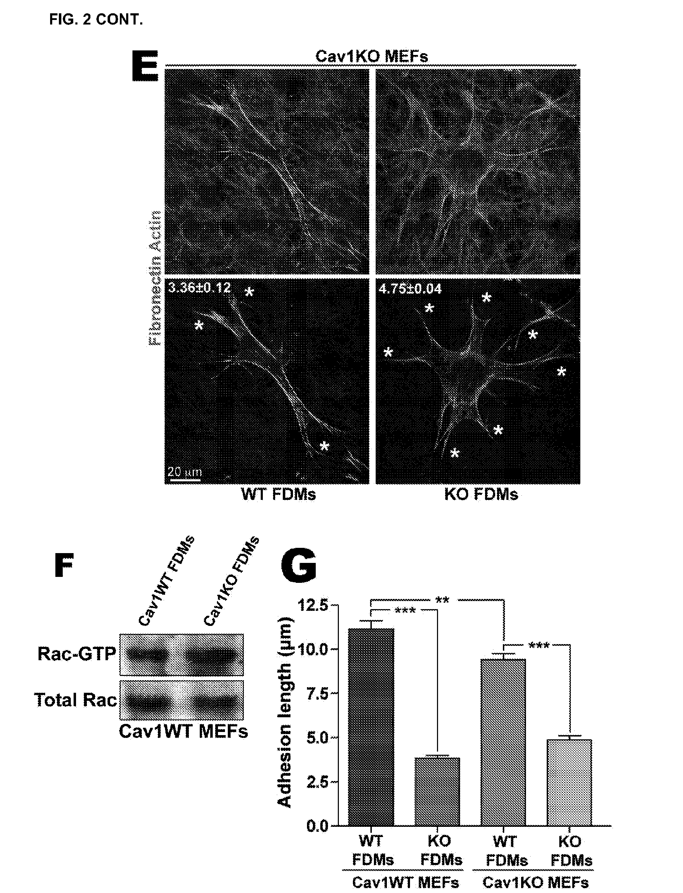

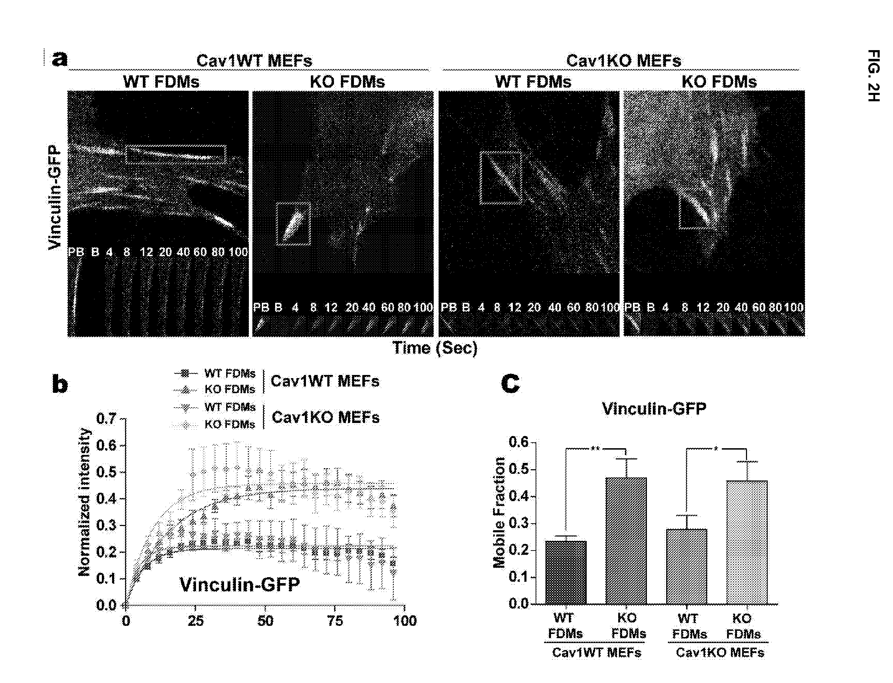

**Figure 2.** Cav1-Dependent Extracellular Environment Regulates Cell Shape, Protrusion Number, Rac1 Activity, and Maturation of Integrin-Dependent Adhesions (A) FDMs were generated from Cav1WT and KO MEFs (or KO MEFs rescued with Cav1WT or CavY14F) and seeded with unmodified or reconstituted MEFs as indicated. (B) EFs of Cav1WT and KO MEFs seeded in the indicated FDMs. (C) EFs of Cav1KO MEFs rescued by re-expression (+) of Cav1WT or CavY14F and seeded in FDMs generated by similarly rescued Cav1KO MEFs. (D) Representative Metamorph masks from experiments as in (C), with calculated EFs. (E) Representative Cav1KO MEFS seeded in Cav1WT or Cav1KO FDMs; average protrusions per cell are shown. (F) Rac-GTP levels and total Rac expression in Cav1WT MEFs seeded in Cav1WT or KO FDMs. (G) Length of integrin-dependent adhesions (indicated by 9EG7 staining). (H) FRAP (Fluorescence Recovery After Photobleaching) analysis of vinculin-GFP (Green Fluorescent Protein)-labeled adhesions in transfected cells seeded in the indicated FDMs. (a) Timelapse sequences showing corresponding regions before photobleaching (PB), immediately after photobleaching (B), and during recovery. (b) Quantification of vinculin-GFP fluorescence recovery for each condition. (c) Percentage of recovery (boxed areas in a) showing the size of the mobile vinculin-GFP fraction. Data are representative of three independent experiments ($6 \leq$ adhesions $\leq 15$). Data are represented as mean $\pm$ standard error of the mean (SEM).

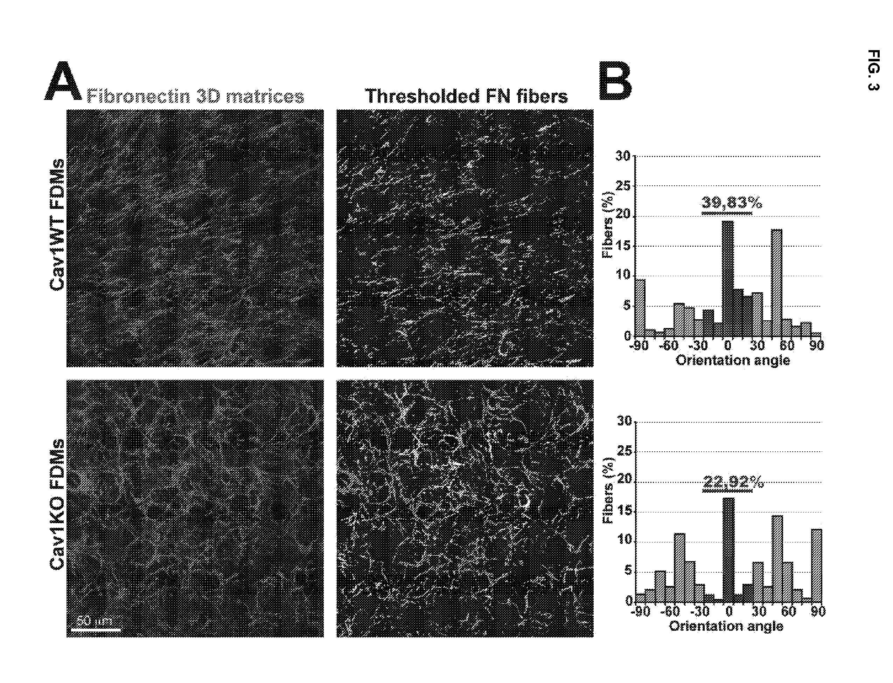

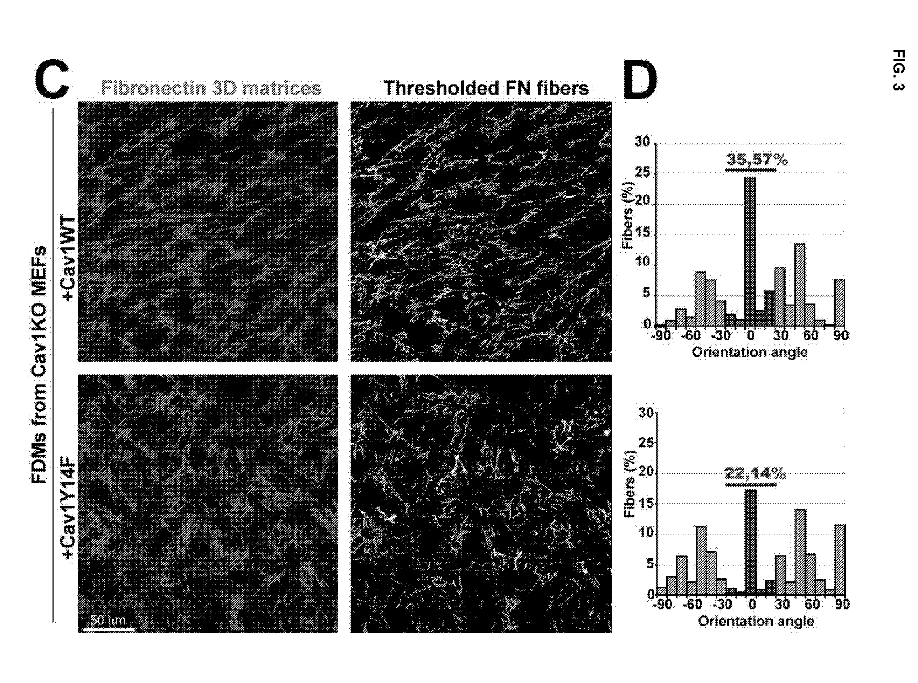

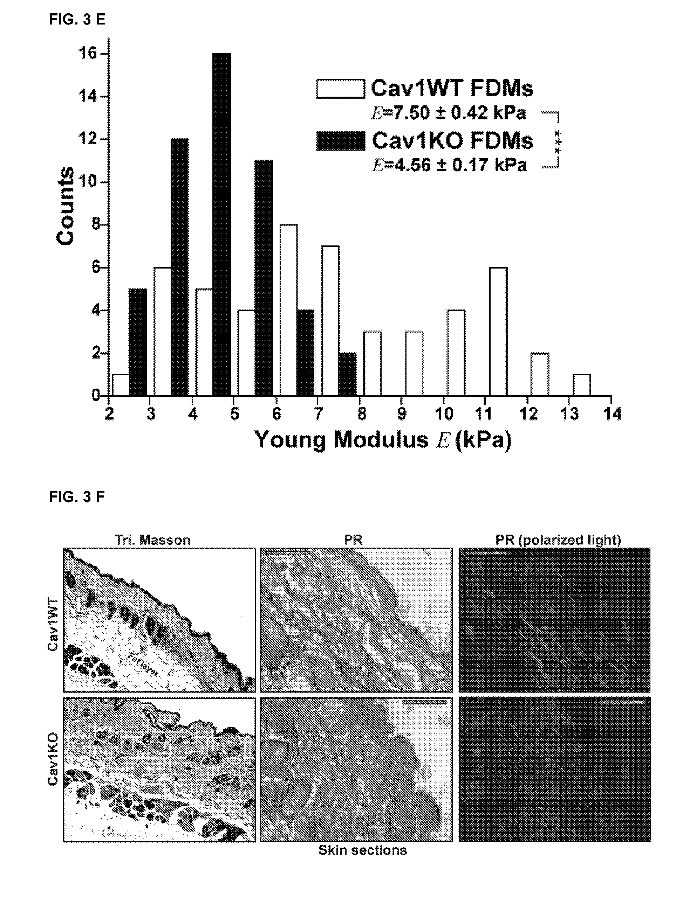

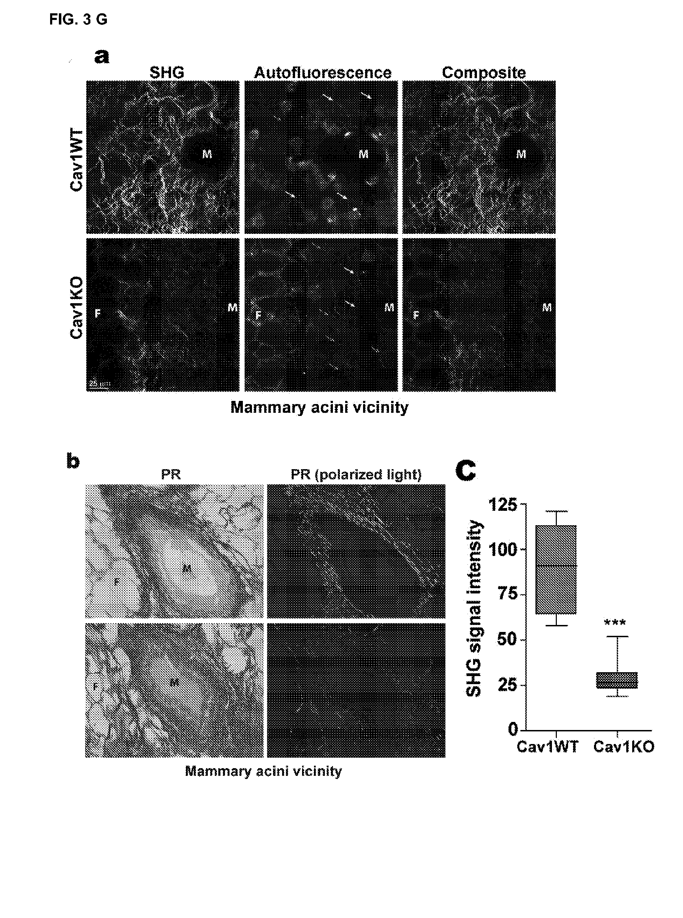

**Figure 3.** Cav1 Promotes Patterning and Stiffness of 3D Matrices and Favors Normal Tissue Architecture (A-D) FDMs were generated and after cell extraction were fixed and labeled. The orientation of all thresholded FN fibers was quantified and plotted against the modal angle (set at 0°). (E) Atomic force microscopy was used to plot point-by-point force versus distance along fibers. The chart shows quantification of Young modulus. (F) Skin sections of WT and Cav1KO mice stained with Masson's trichrome and picrosirius red (PR). Polarized light highlights fibrillar collagen. (G) Stromal organization in WT and Cav1KO mammary gland. (a) Multiphoton excitation microscopy coupled to second harmomic generation imaging (MPESHG) of intact fixed glands; SHG and autofluorescence signals are shown. Red and yellow arrows mark curled and straight collagen fibers devoid of SHG signal. (b) Mammary gland sections from WT and Cav1KO mice stained as in (F). M = mammary gland, F = fat cell. (c) Quantification of SHG signal intensity as in (a). Data are represented as mean $\pm$ standard error of the mean (SEM).

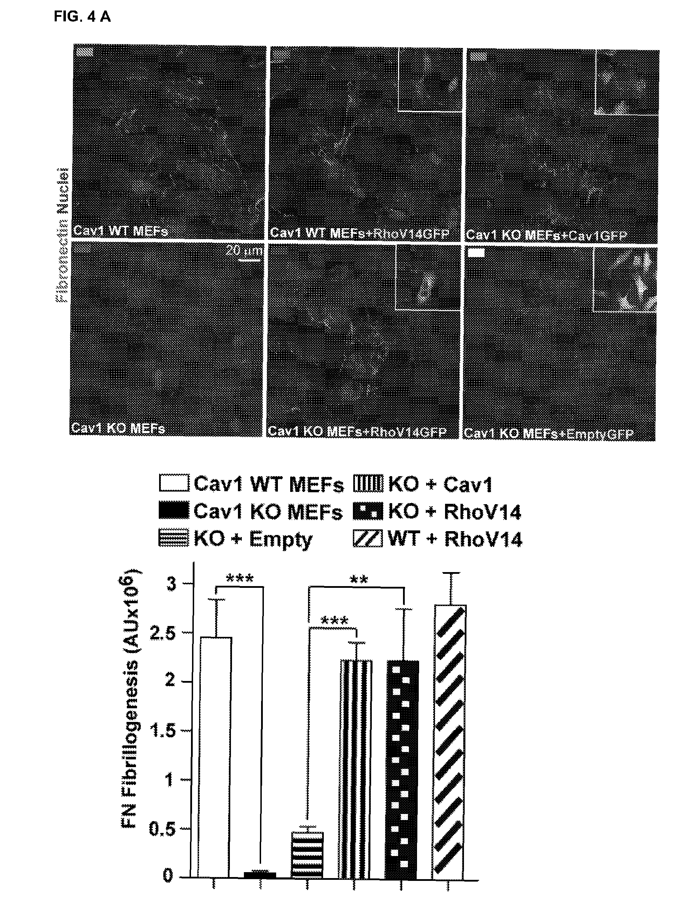

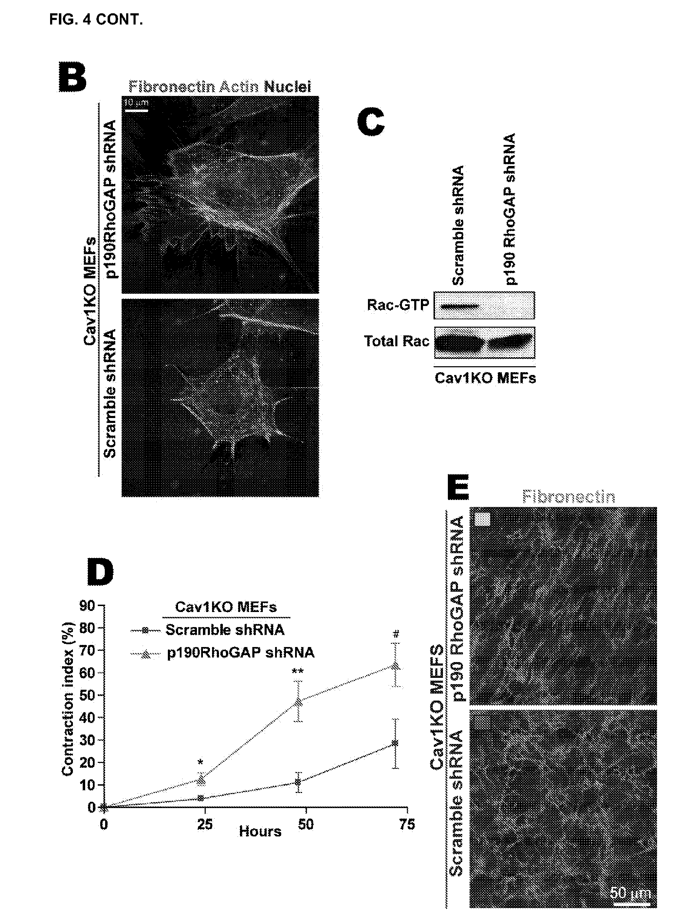

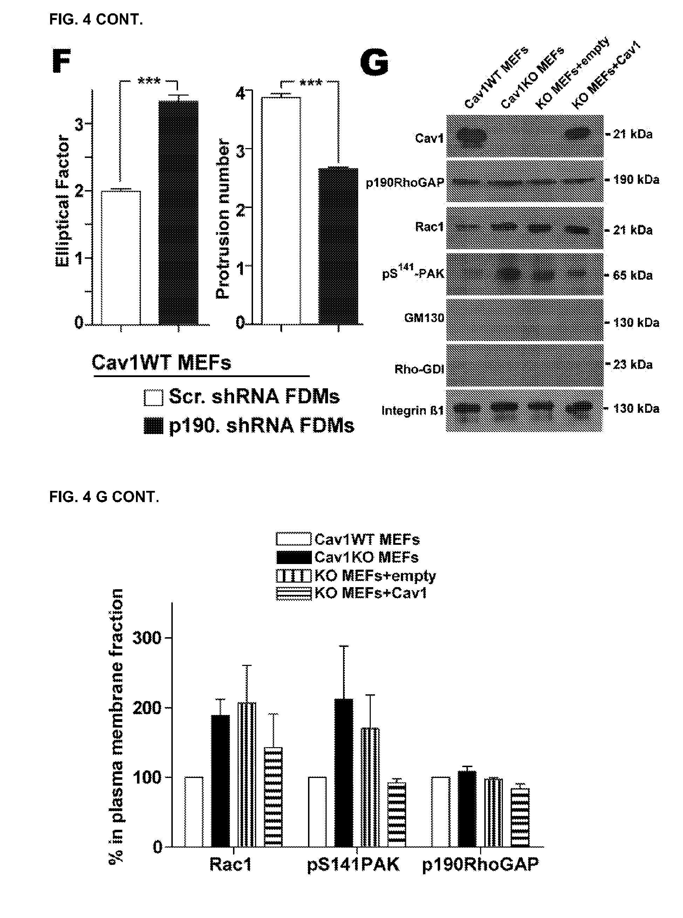

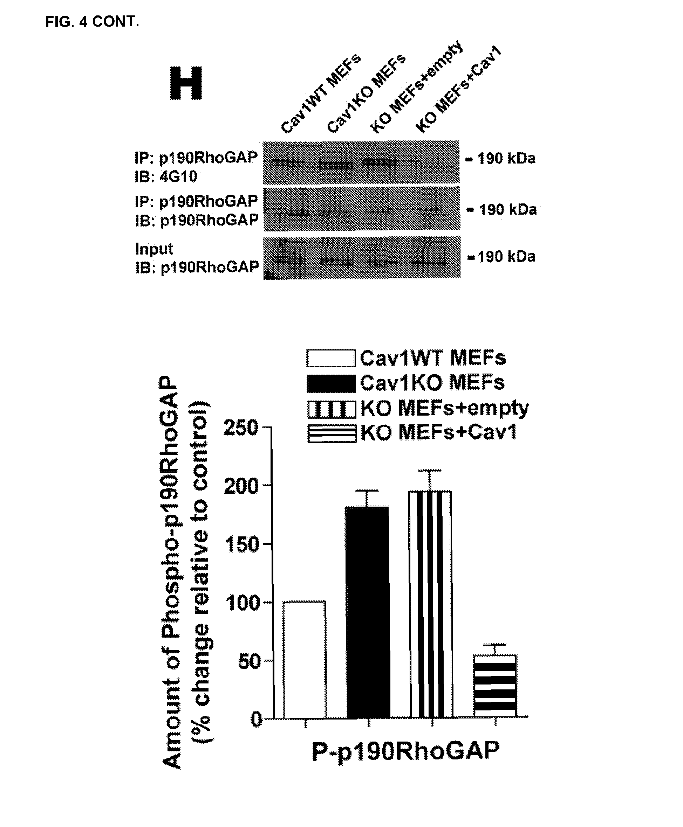

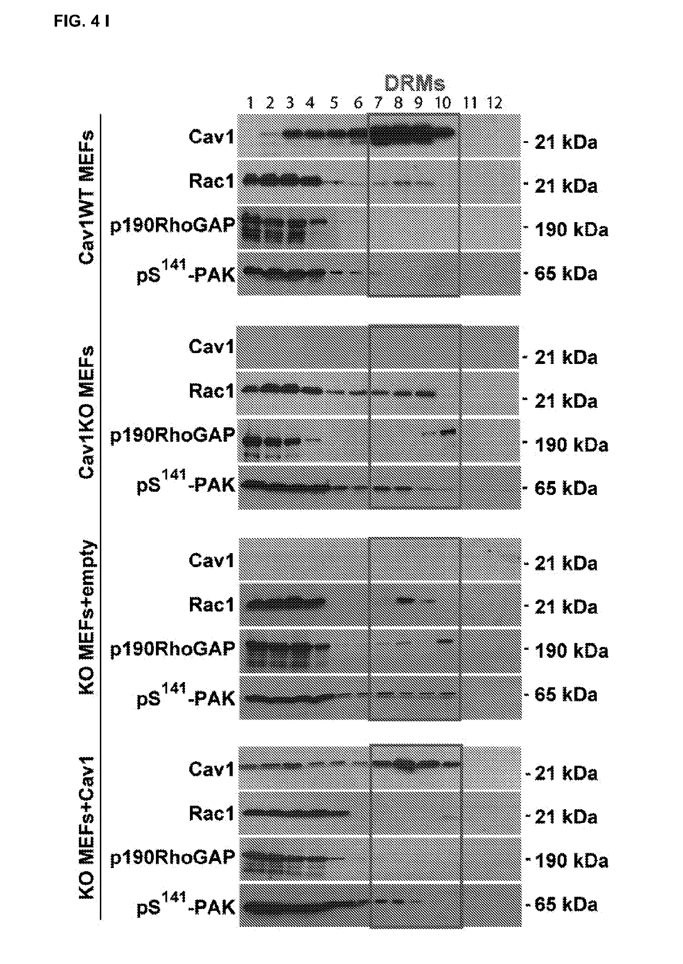

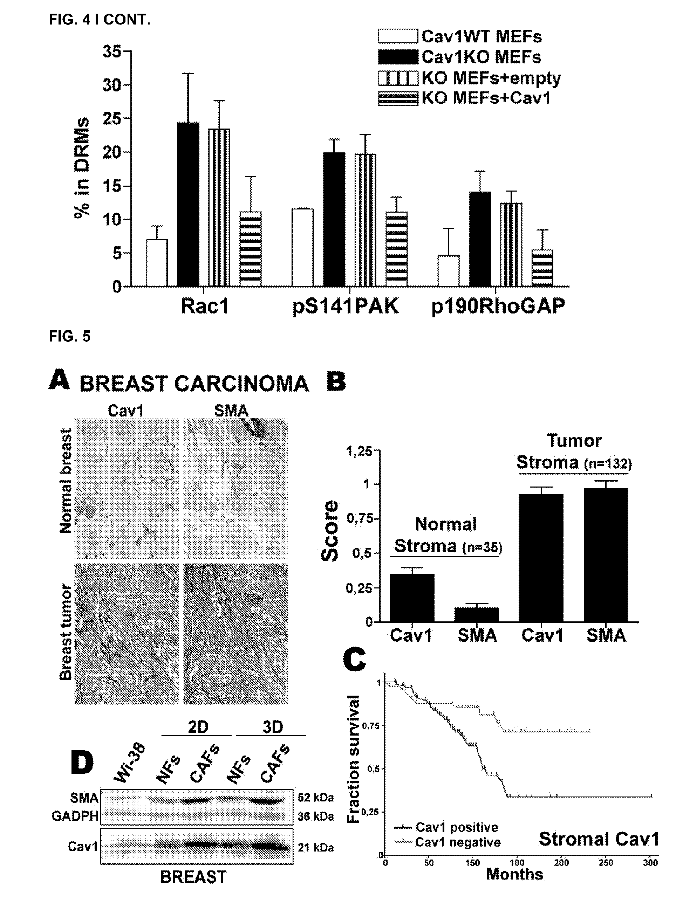

**Figure 4.** Cav1 Promotes Force-Dependent Microenvironment Remodeling via Rho GTPase Activation (A) Indicated MEFs expressing GFP-tagged WT Cav1, RhoV14, or empty vector (see immunoblot) were plated on FN (24 hr). FN remodeling was quantified by thresholding for bright FN fibrils. (B) Cav1KO MEFs stably expressing scrambled or p190 shRNA were plated as in (A) and analyzed for (C) Rac1-GTPase activity and (D) Col-I gel contraction. (E) FN staining of FDMs generated from the indicated MEFs. (F) EF (left) and average protrusion number (right) of Cav1WT MEFs seeded on FDMs generated as in (E). (G) Immunoblot analysis of purified PM fractions from the indicated MEFs. Quantified and protein levels are plotted relative to Cav1WT MEFs. (H) p190 was immunoprecipitated from samples prepared as in (G) and probed for p190 and phosphotyrosine (4G10). Chart shows quantification of phosphorylated p190. (I) The indicated MEFs were fractioned, and immunoblot signals in DRM (Detergent-resistant membranes) fractions quantified and plotted. Data are represented as mean $\pm$ standard error of the mean (SEM).

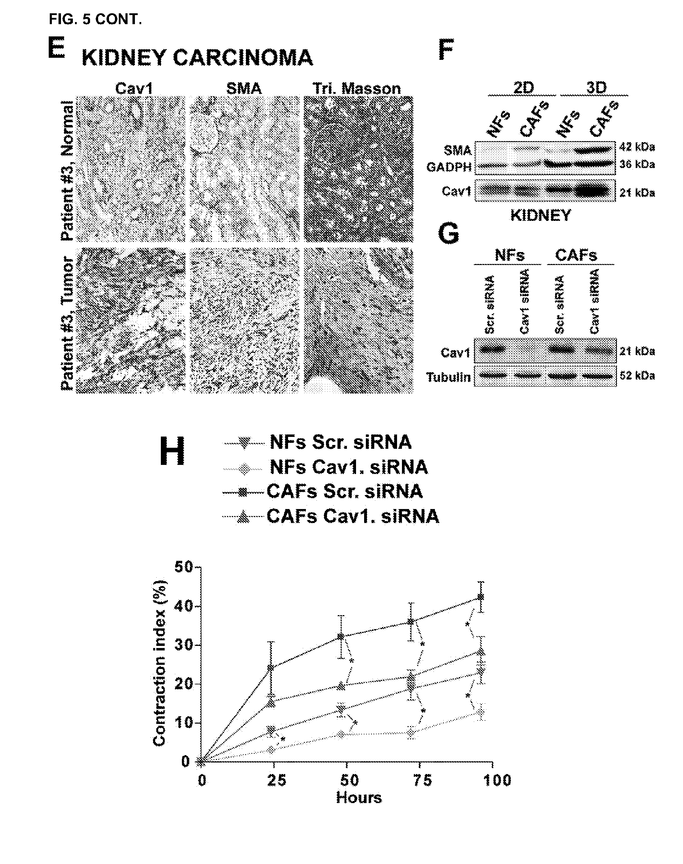

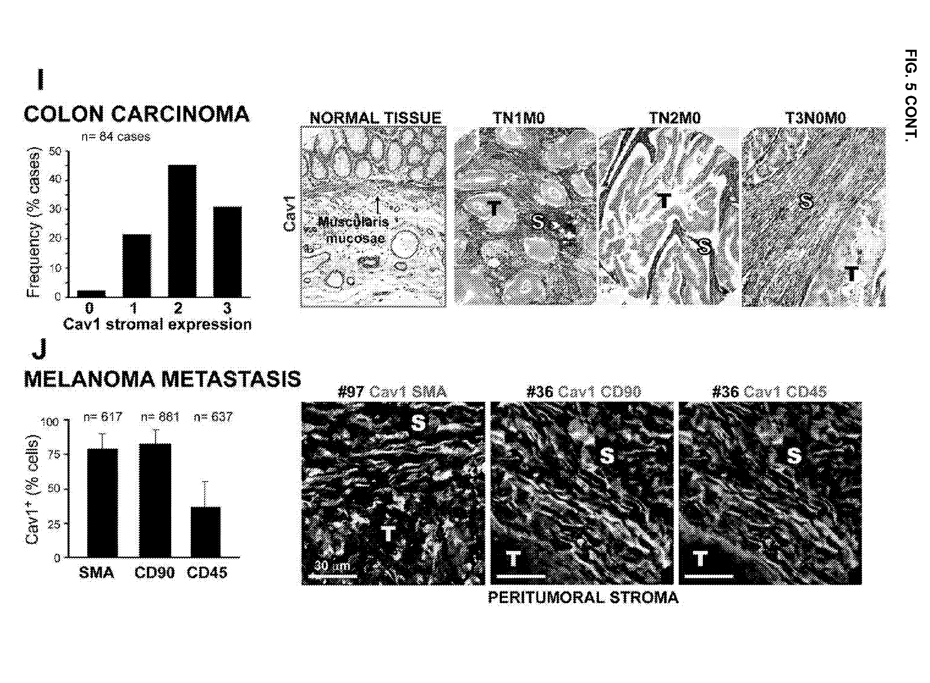

**Figure 5.** Stroma of Human Breast, Kidney, and Colon Carcinomas and Melanoma Metastases Are Enriched in Cav1-Expressing Fibroblasts (A) Representative images of normal and breast cancer tissue stained for Cav1 and SMA. (B) Staining scores for Cav1 and SMA in normal (n = 35) and tumor (n = 132) tissues. (C) Kaplan-meier curve of progression-free survival for patients sorted by stromal Cav1 expression.

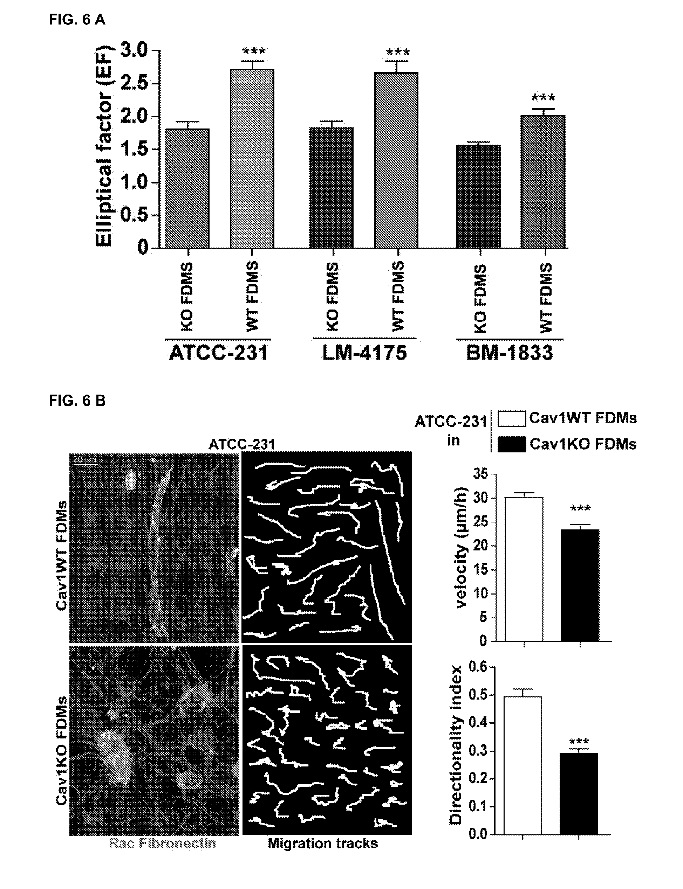

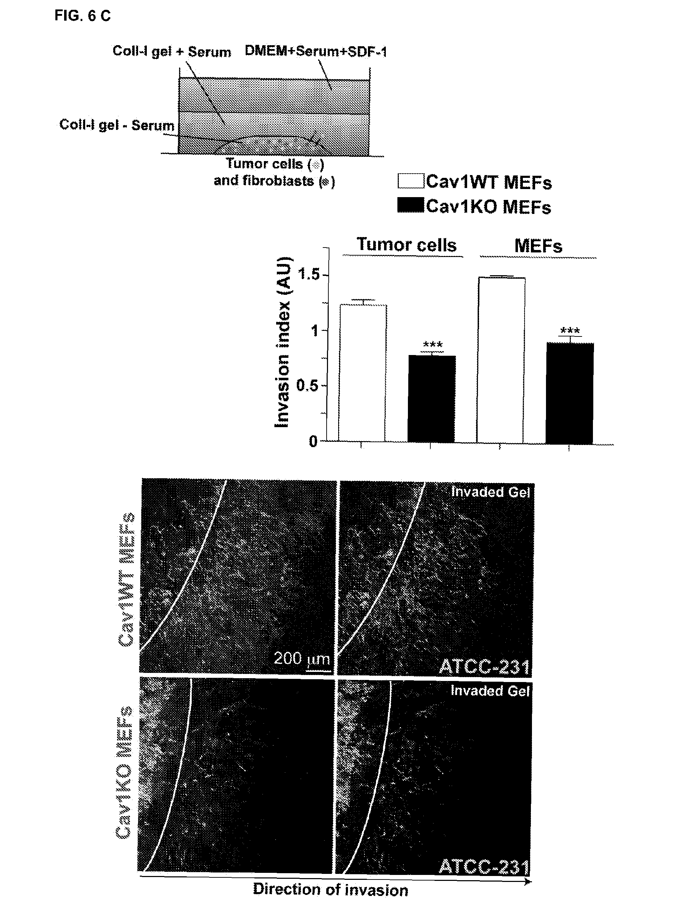

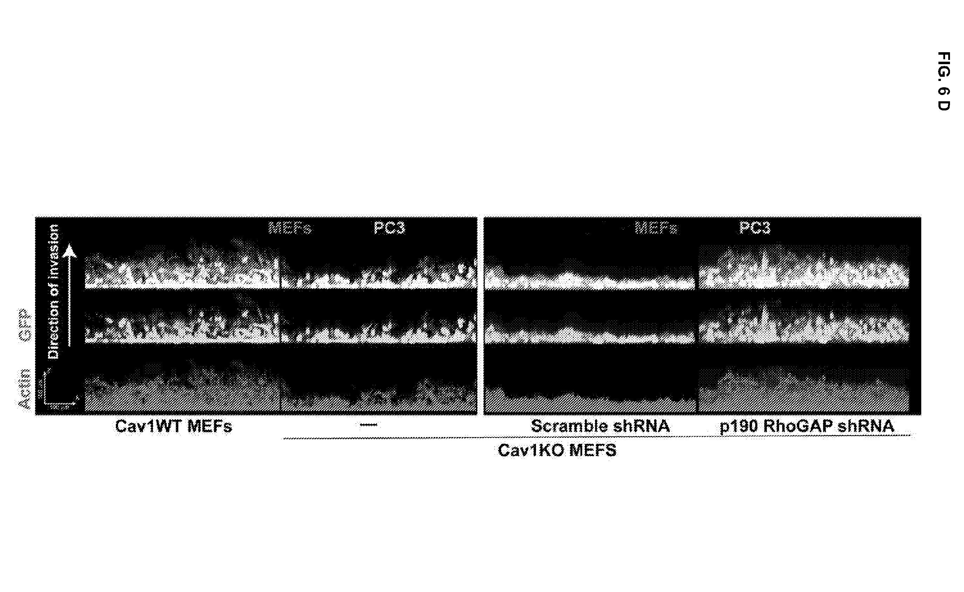

**Figure 6.** Cav1-Dependent 3D Microenvironment Stimulates TC Migration and Invasion *In Vitro* (A) EFs of ATCC-231, LM-4175, and BM-1833 metastatic cells seeded in Cav1WT or Cav1KO FDMs. (B) ATCC-231 cells grown in the indicated FDMs (6 hr) were monitored for 12 hr. Samples were labeled for Rac and FN to reveal cell morphology and FDM structure. Example migration tracks are depicted. Charts show quantification of cell velocity and directionality. (C) Col-I gels containing prelabeled TCs (tumor cells) and MEFs were placed under cell-free gel (scheme) and 3D TC invasion was quantified. Actin staining allowed distinction of MEFs from TCs. (D) GFP-expressing PC3 prostate TCs were seeded in Matrigel with the indicated MEFs and cultured for 6 days. Cells were fixed and stained as indicated. Data are represented as mean ± standard error of the mean (SEM).

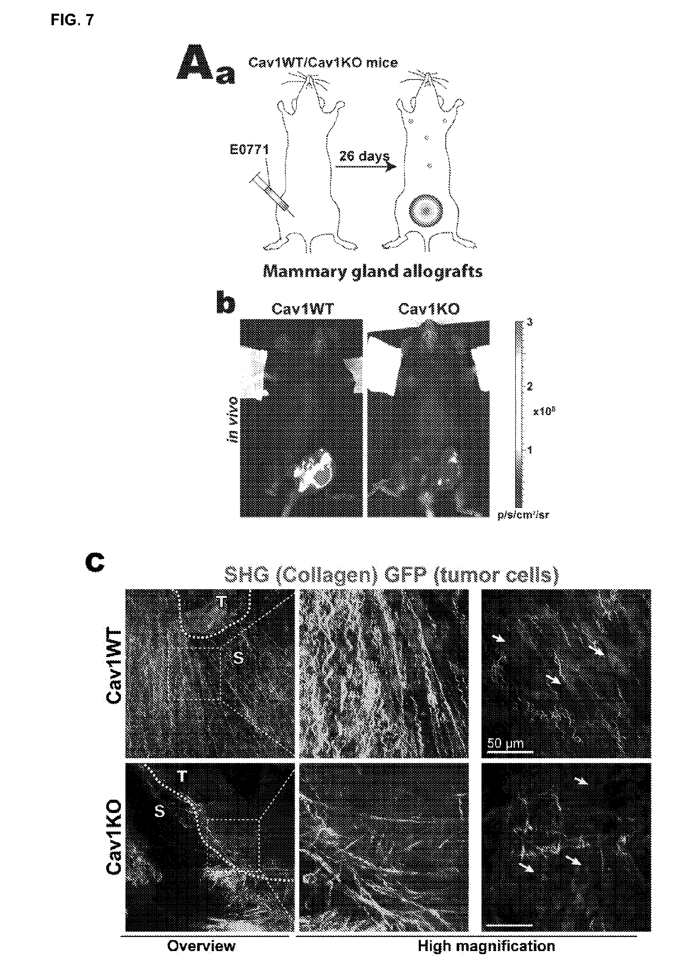

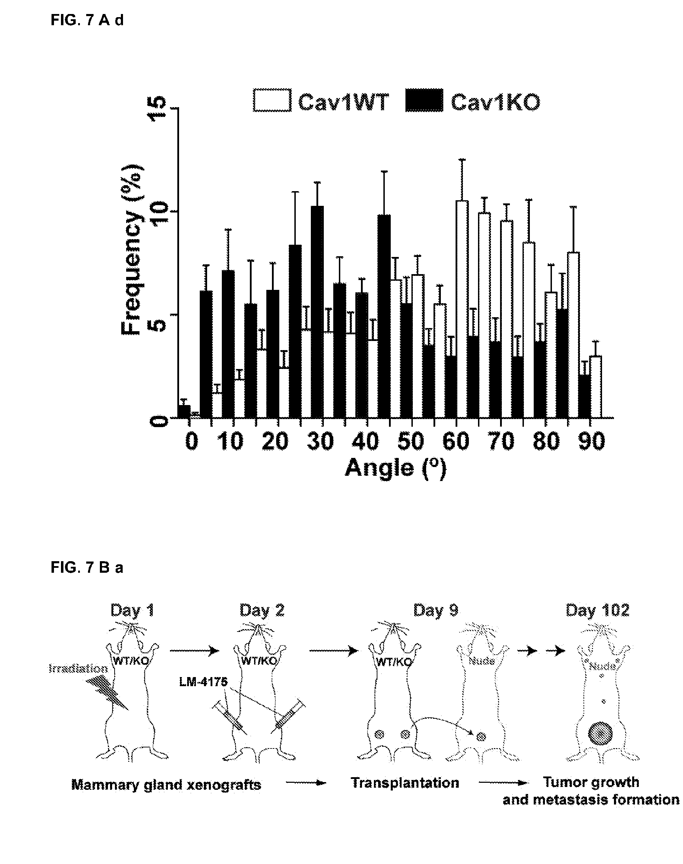

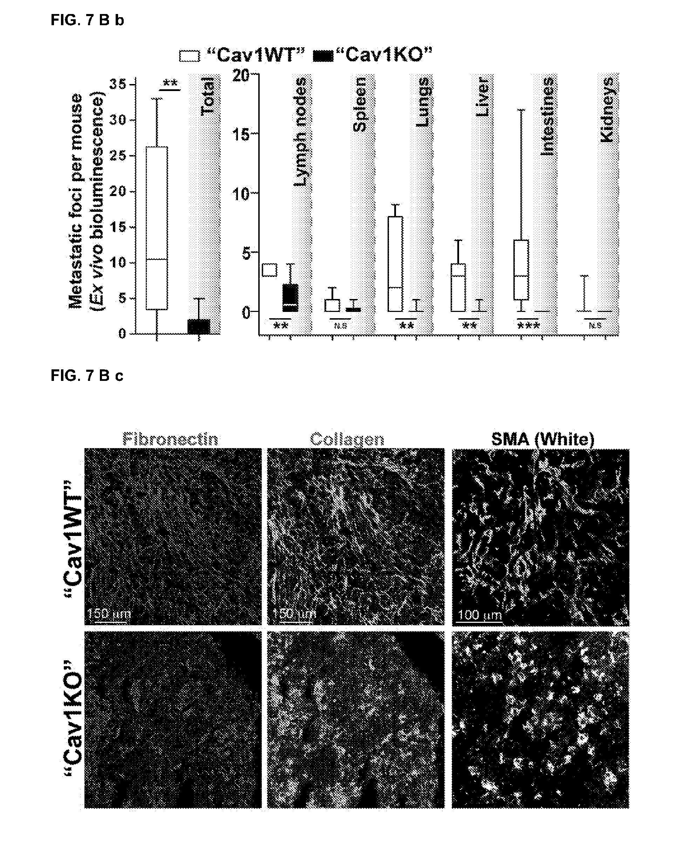

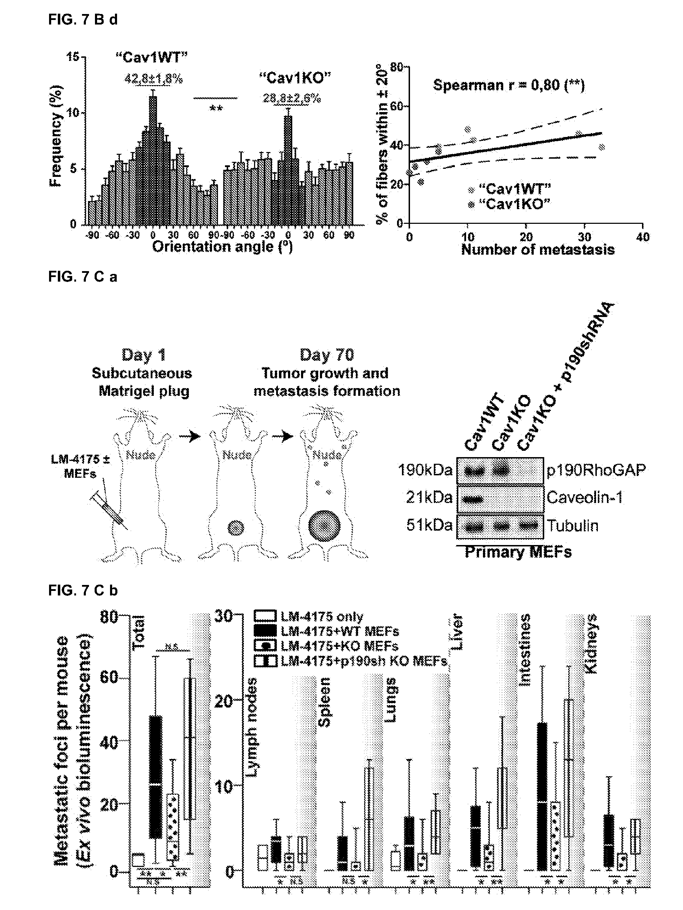

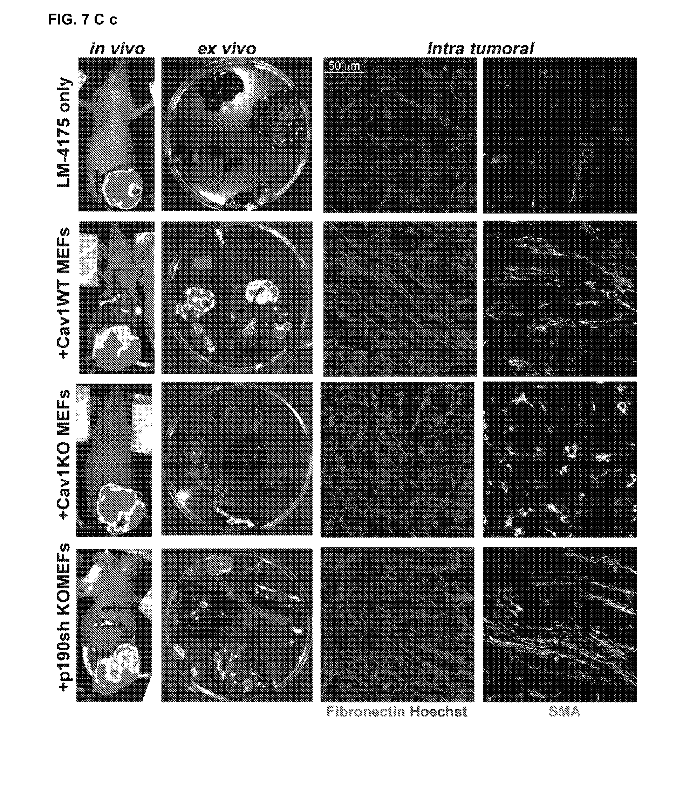

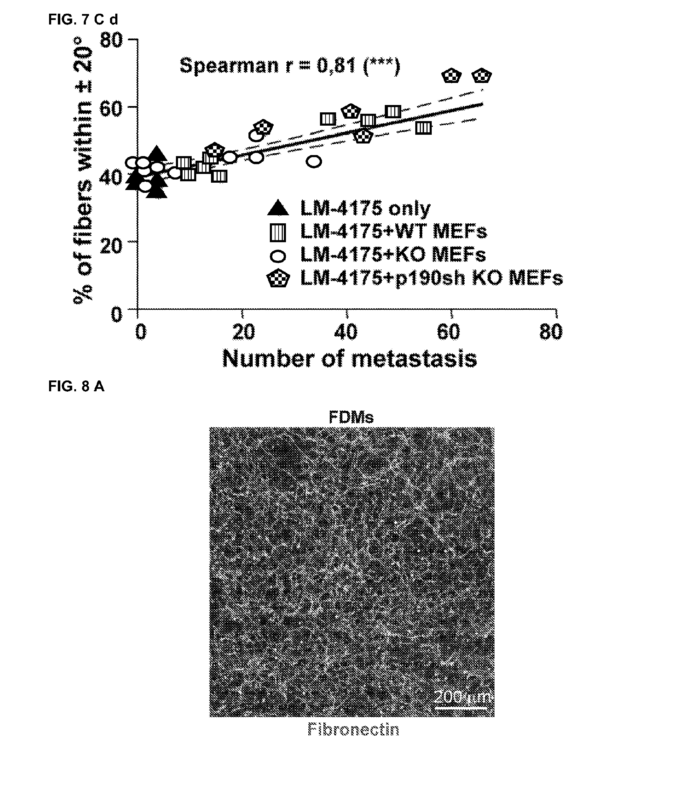

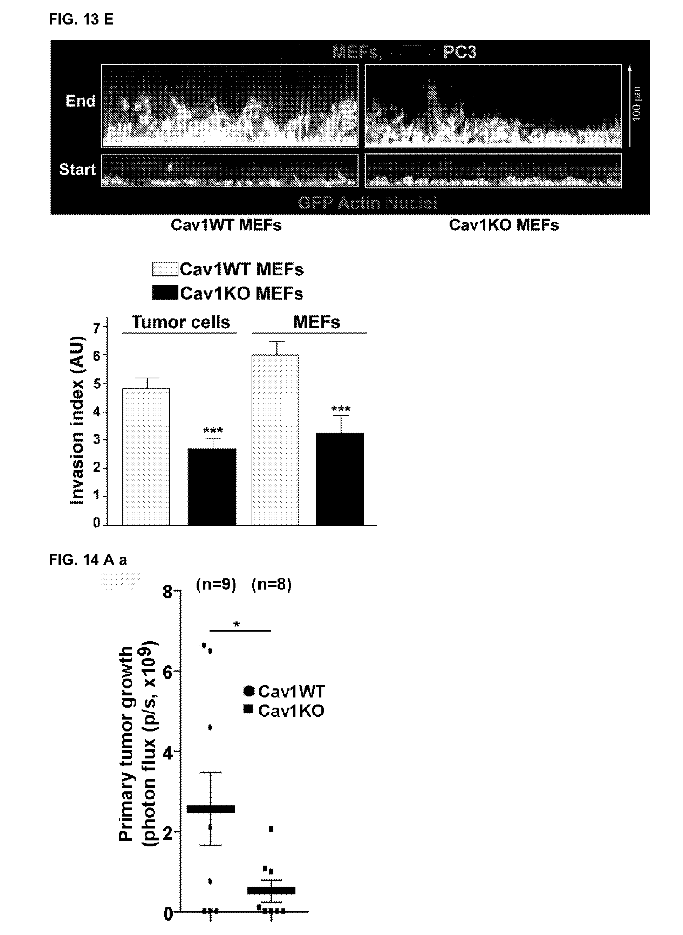

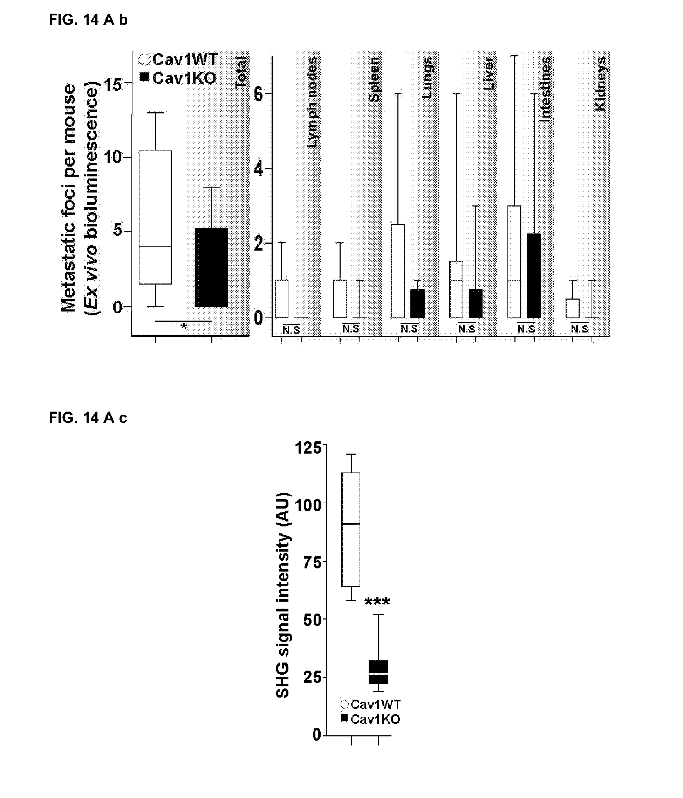

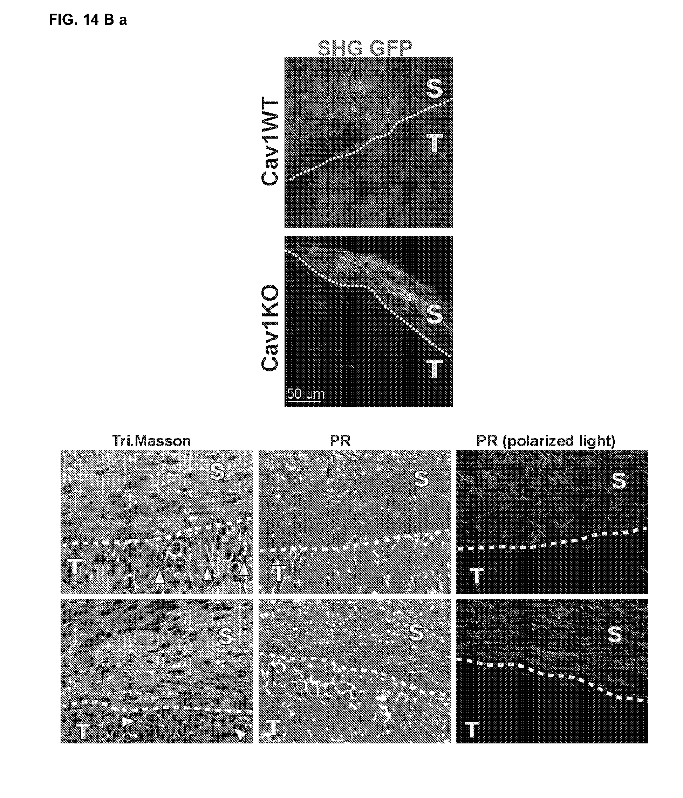

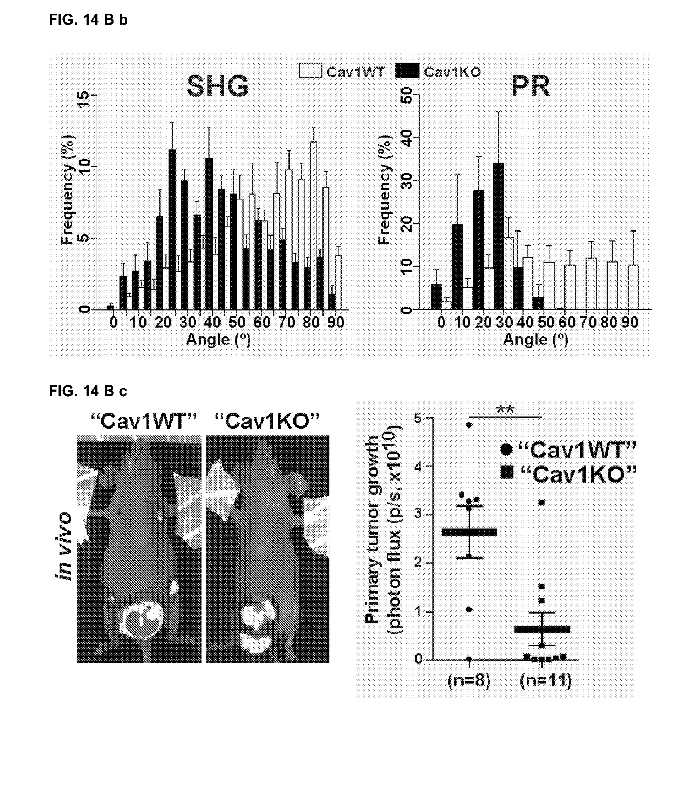

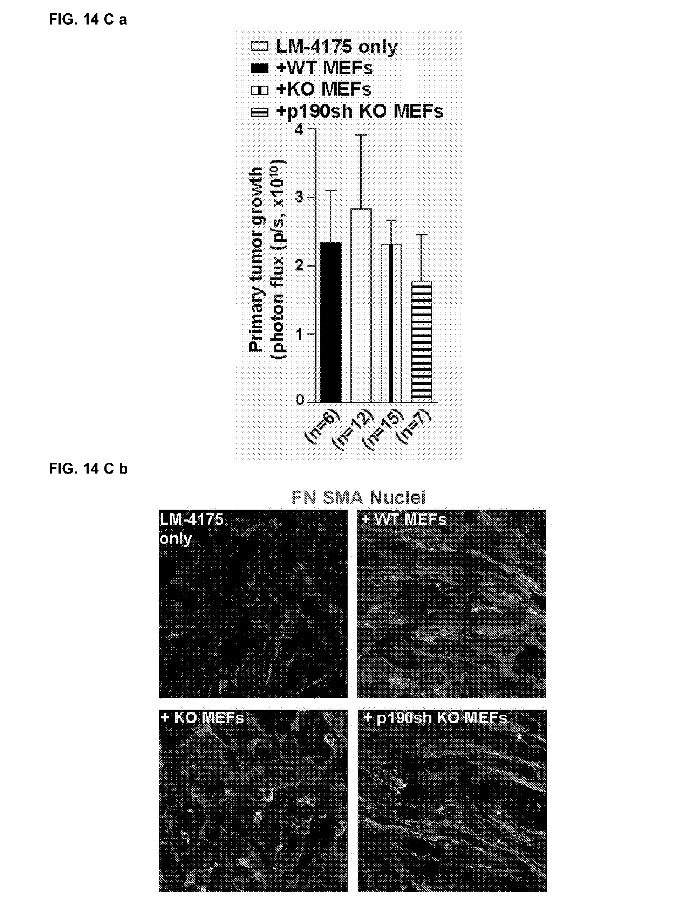

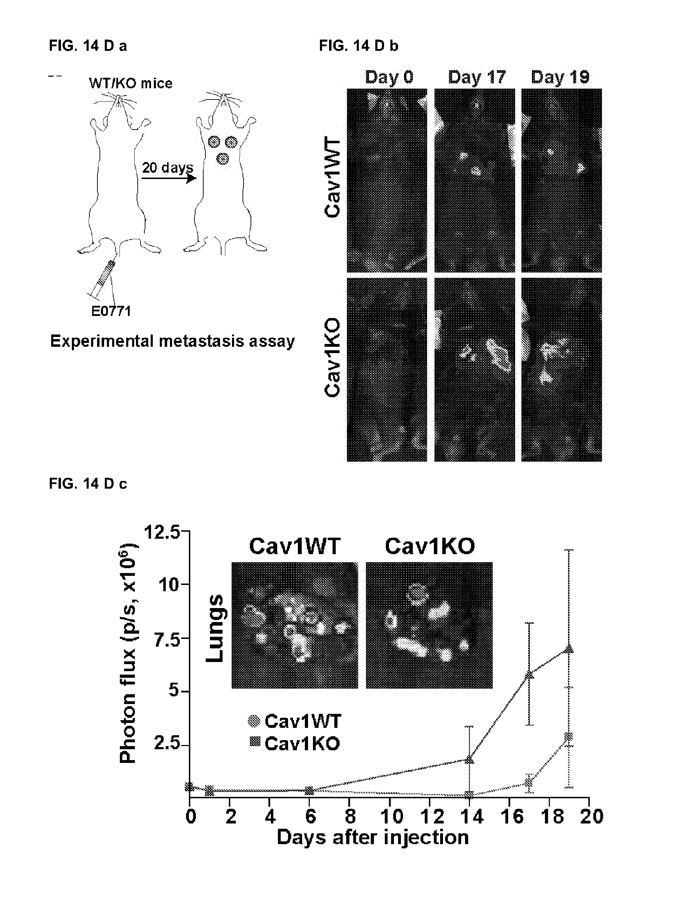

**Figure 7.** Cav1-Dependent 3D Microenvironment Stimulates *In Vivo* Tumor Cell Invasion and Increases Metastatic Potency. (A) Orthotopic mammary gland allografts. (a) Experimental scheme. (b) Bioluminescence detection of TCs (representative images). Scale depicts the photon flux (photons per second). (c) MPE-SHG of primary tumor explants. Arrows mark invading and encapsulated TCs. (d) Quantification of the angle of collagen fibers to the tumor boundary by SHG. (B) Orthotopic mammary gland xenografts and tumor transplantation in nude mice. (a) Experimental scheme. *Ex vivo* quantification of the distribution of total and organ-specific metastatic foci (right). (c) Immunostaining of extracted tumors as indicated. (d) Quantification of intratumoral orientation of FN fibers plotted against the modal angle (set at 0°, left). Correlation analysis (right). (C) Subcutaneous Matrigel injection of LM-4175 TCs plus the indicated pMEFs. (a) Experimental scheme. Immunoblot shows efficiency of p190 silencing and Cav1 expression. (b) Distribution of total and individual metastatic foci (*ex vivo*, day 70). (c) Representative images of primary tumors *in vivo*, extracted organs ex *vivo*, and immunostained tumors. (d) Correlation analysis. Data are represented as mean ± standard error of the mean (SEM). See also Figure 14.

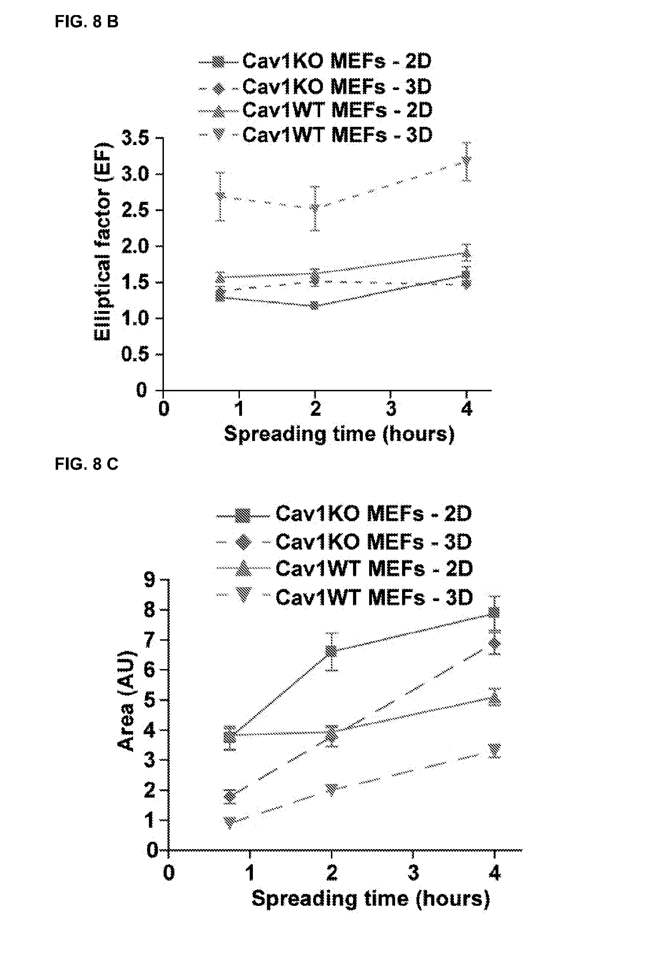

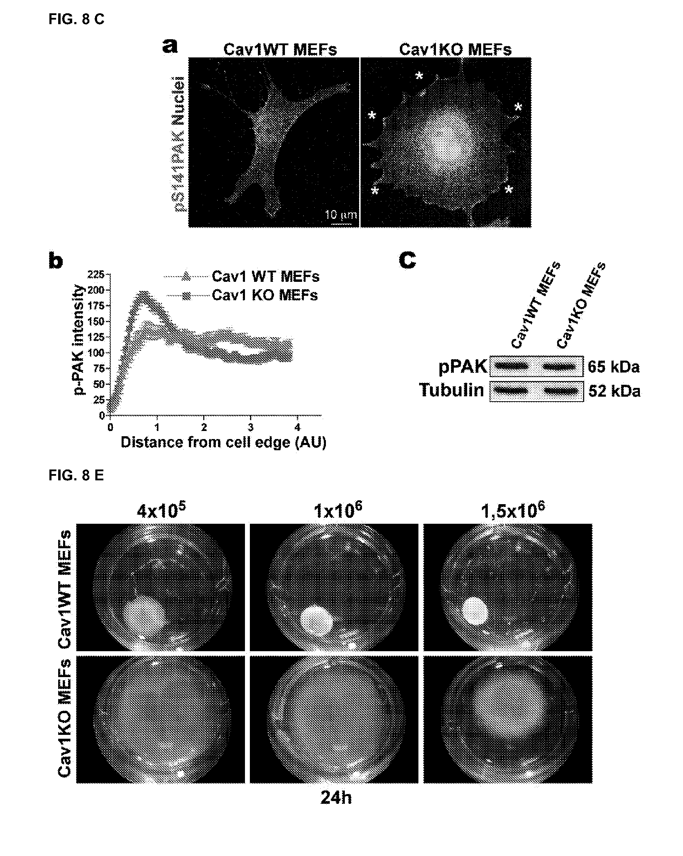

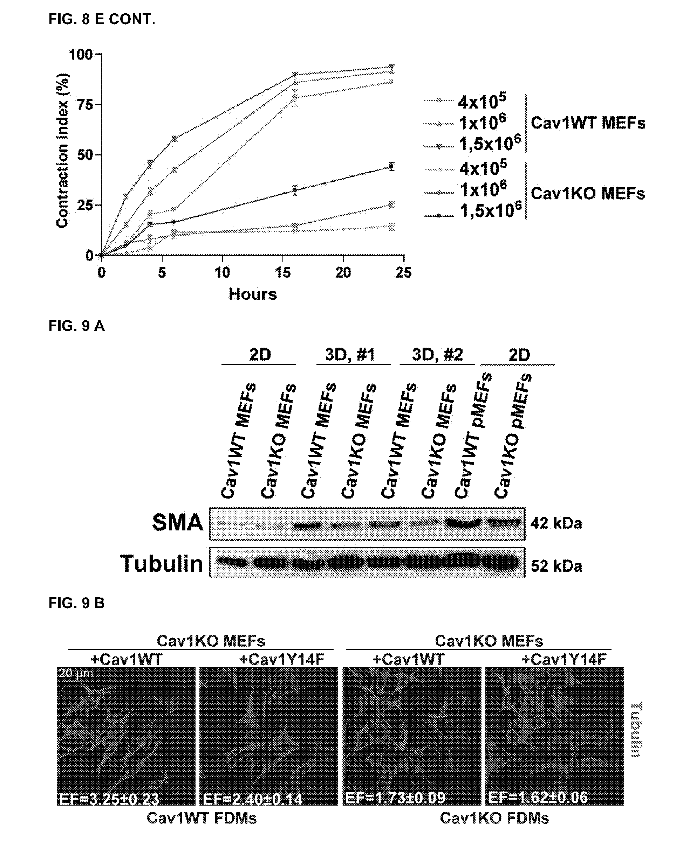

**Figura 8.** (A) Example FDM labeled for FN. Cav1WT and Cav1KO MEFs were plated for 4 hr on NIH 3T3-derived FDMs (3D) or on soluble FN (5 mg/ml) (2D). The charts show the evolution of EF (B) and cell-occupied area (C) after plating. (D) Cav1WT and Cav1KO MEFs were plated for 4 hr on FN (5 mg/ml). (a) Cells were labeled for Rac1-activated phospho-S141PAK (pPAK) and counterstained with Hoechst; pPAK concentrates at the edge of Cav1 KO MEFs (asterisks). (b) Pixel intensity for pPAK was measured from at the cell edge. (c) Immunoblot showing pPAK levels. Tubulin was used as loading control. (E) Effect of cell number on collagen gel contraction by Cav1WT and Cav1KO MEFs. Data are represented as mean ± standard error of the mean (SEM).

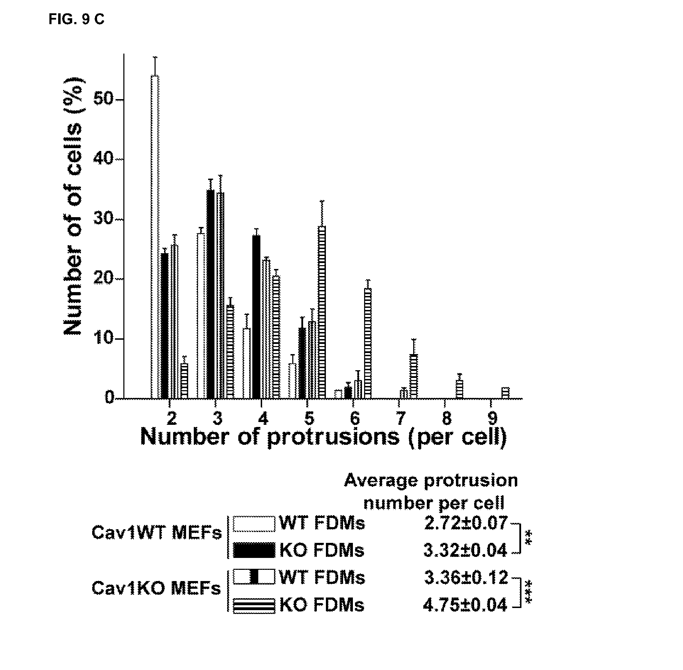

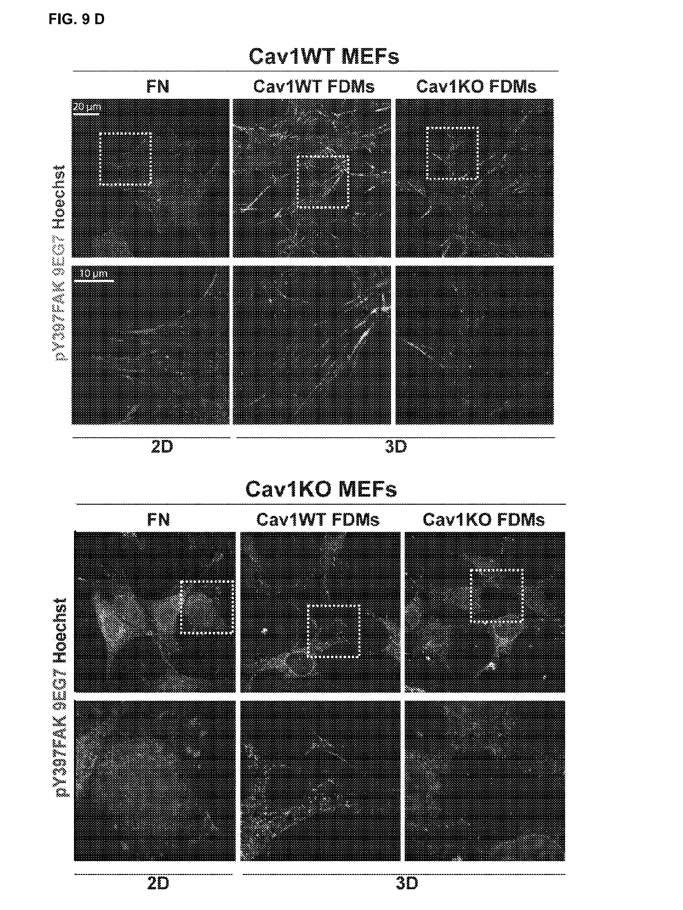

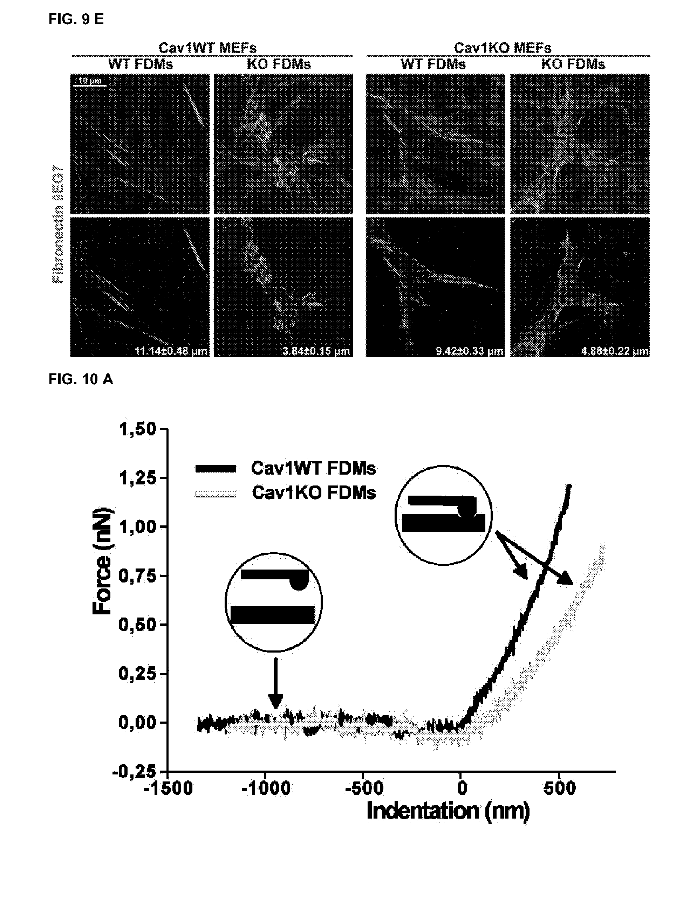

**Figura 9.** (A) SMA expression by Cav1WT and KO MEFs, both immortalized and primary (pMEFs), cultured under the indicated conditions. (B) Representative images of tubulin staining and calculated EFs for Cav1KO MEFs rescued with either WT or Y14F Cav1 and plated for 6 hr on the indicated FDMs. (C) Quantification of total and average numbers of protrusions per cell in samples depicted in (B). (D) Cav1WT and Cav1KO MEFs were grown on soluble FN (2D) or on Cav1WT- or Cav1KO-derived FDMs (3D). Fixed cells were stained for active integrins (9EG7), pY397FAK, and nuclei (Hoechst). Boxed areas in the top row are shown in magnified view in the lower panels. (E) Cav1WT and KO MEFs plated on the indicated FDMs were fixed and labeled for FN and active integrins (9EG7). Numbers indicate the average length of integrin-dependent adhesions. Data are represented as mean ± standard error of the mean (SEM).

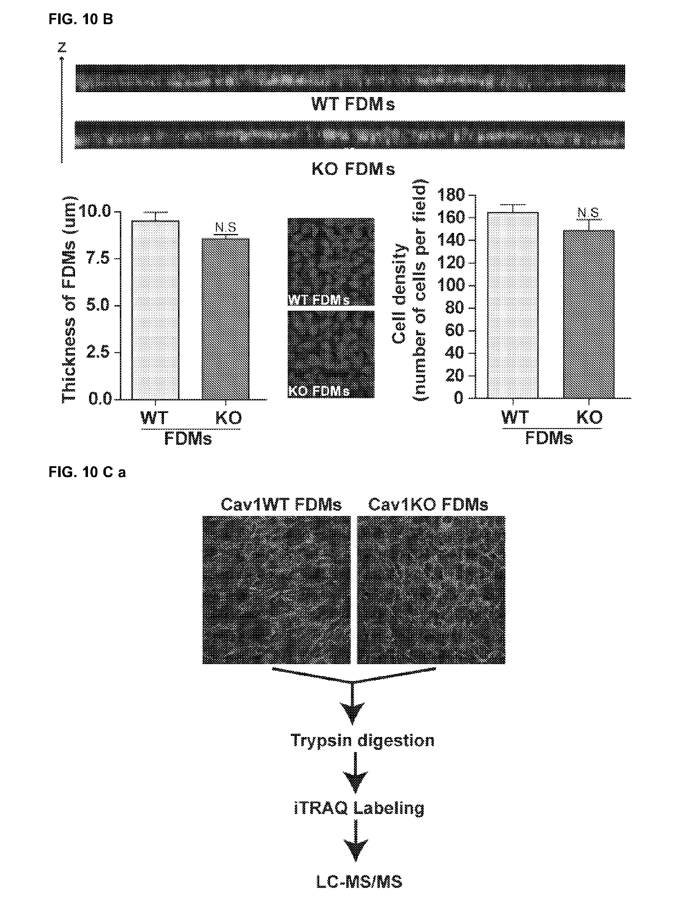

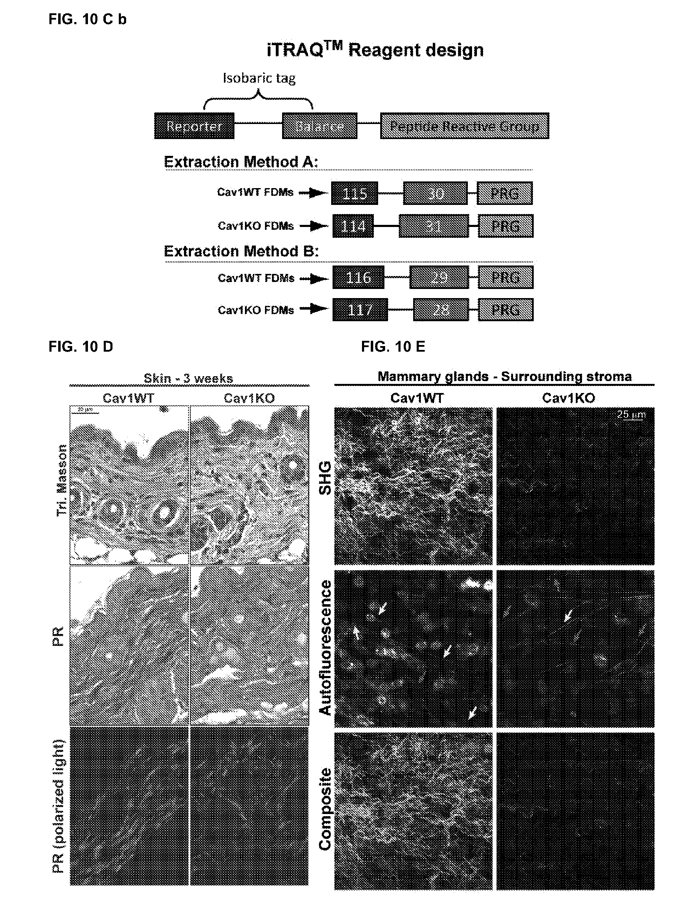

**Figura 10.** (A) Representative force curves from Cav1WT FDMs and Cav1KO FDMs. Curves overlap when the tip is at a distance from the fiber, but once the tip establishes mechanical contact with the fiber the curves diverge. To achieve the same force with both FDMs, the tip needs to push the Cav1KO FDMs harder, and consequently the gradient is shallower. (B) Matrix thickness and cell density in unextracted FDMs generated by Cav1WT and KO MEFs. Representative images show cultures stained for FN and nuclei. (C) Identification and quantification of ITRAQ (Isobaric tag for relative and absolute quantitation)-labeled ECM proteins by liquid chromatography (LC) coupled to tandem mass spectrometry (MS/MS). (a) Experimental design. (b) Design of iTRAQ reagents and FDM labeling by methods A and B (Experimental Procedures). (D) Skin sections of 3-week-old WT and Cav1KO mice stained with Masson's trichrome and picrosirius red (PR). Polarized (orthogonal) light highlights fibrillar collagen. (E) Multiphoton excitation microscopy of intact fixed mammary glands from Cav1WT and KO mice. Images show second harmonic generation (SHG) and autofluorescence signals of surrounding stroma. Arrows mark curly and straight collagen fibers devoid of SHG signal. Data are represented as mean ± standard error of the mean (SEM).

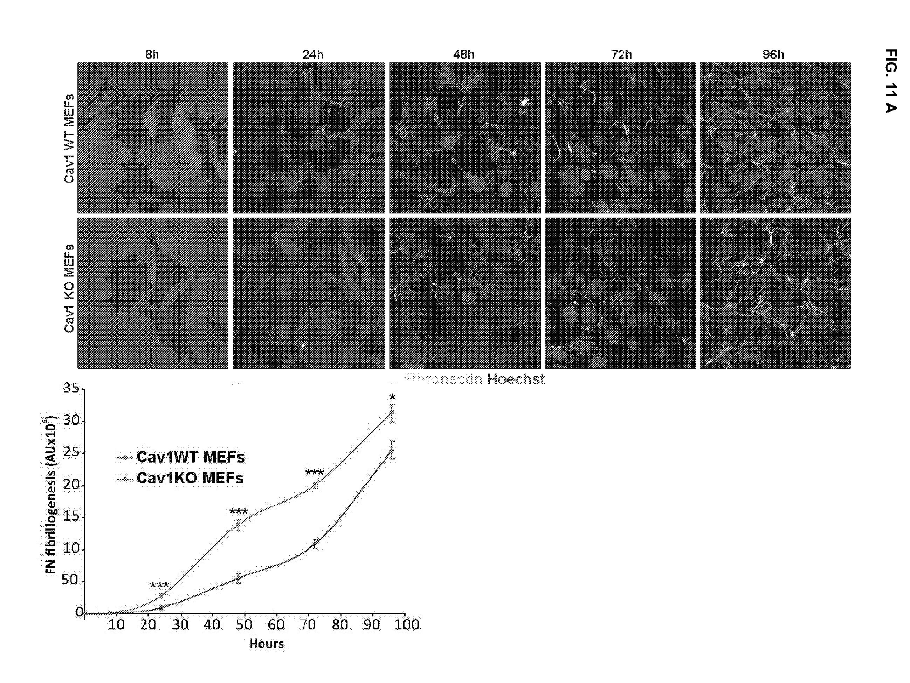

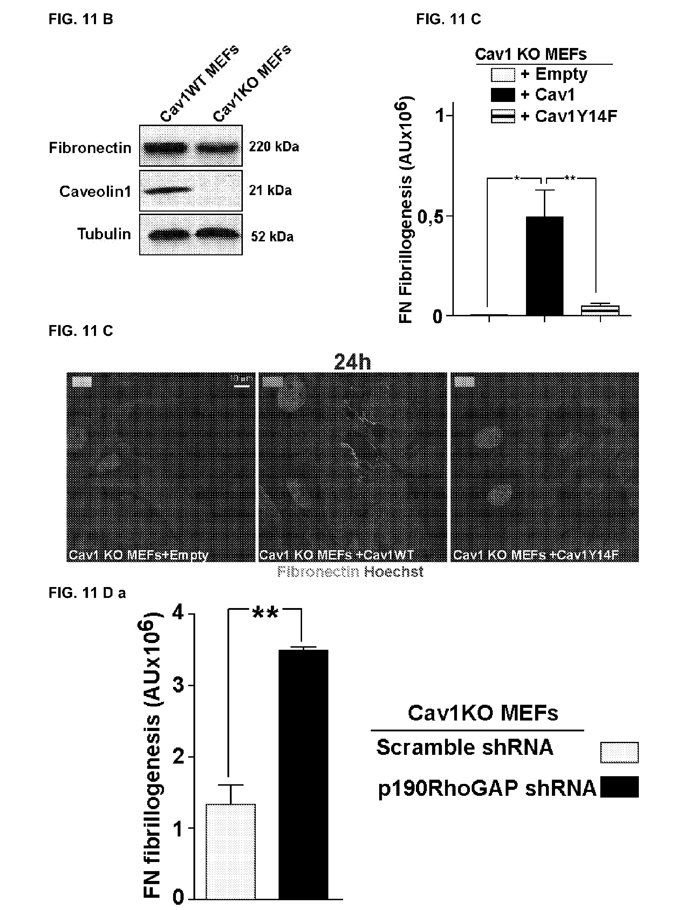

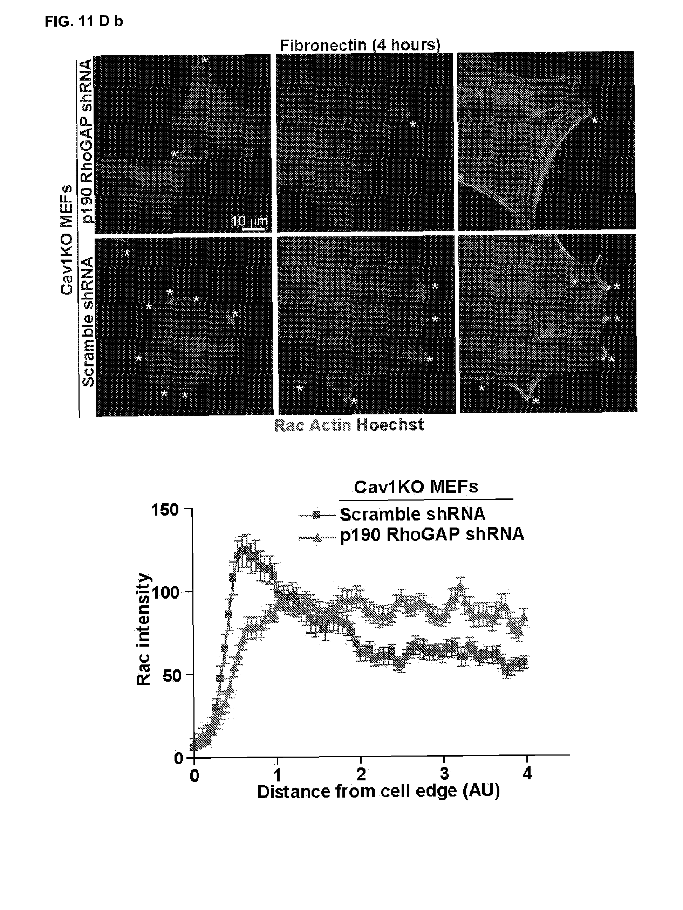

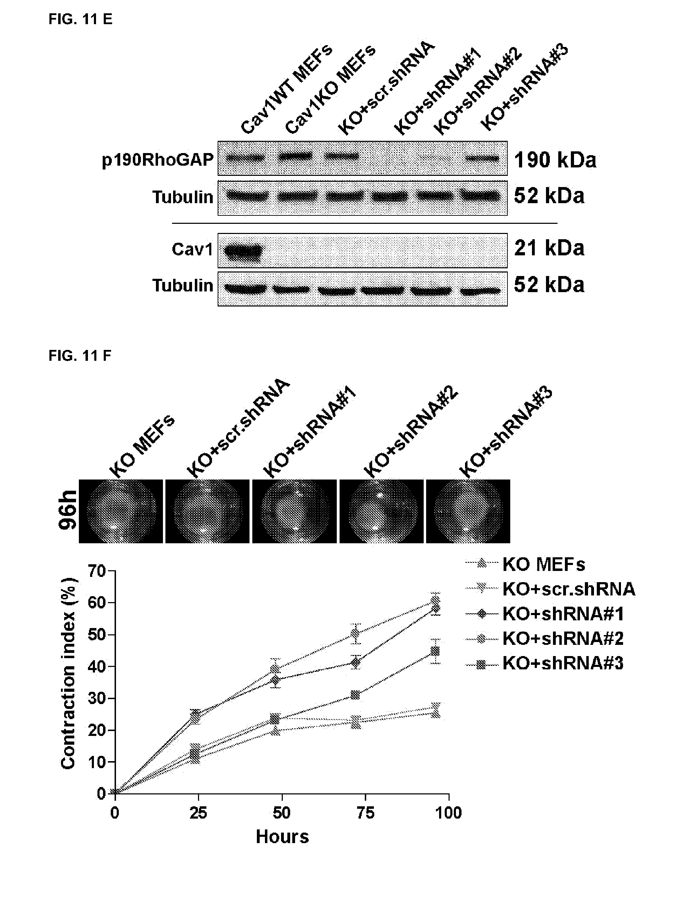

**Figura 11.** (A) Staining for FN and Hoechst in cultures of Cav1WT and KO MEFs grown on FN-gels (3D cultures) for the indicated times. Remodeling of soluble FN is visible as the formation of bright FN fibrils, leaving clear areas depleted of FN stain. The chart tracks FN fibrillogenesis (AU: Arbitrary Units) over time. (B) FN expression in 2D cultures of Cav1WT and KO MEFs grown without serum. The immunoblot shows total expression. Tubulin was used as loading control. (C) FN fibrillogenesis of Cav1KO MEFs stably expressing WT or Y14F Cav1. The chart shows quantification (AU) and the panels display representative images. (D) Effect of stable depletion of p190RhoGAP in Cav1KO MEFs. (a) Quantification of FN fibrillogenesis in Cav1KO MEFs stably expressing p190RhoGAP shRNA sequence#1 (SEQ ID NO: 3). (b) Rac1 membrane distribution. Representative images highlight the loss of strong Rac intensity at the plasma membrane of p190RhoGAP-silenced cells (asterisks). Graph shows Rac1 pixel intensity from the cell edge to the nucleus. (E) Immunoblot showing the effectiveness of three independent p190RhoGAP shRNA sequences on p190RhoGAP expression in Cav1 KO MEFs. Cav1 and tubulin blots are shown as internal controls. (F) Effect of the three p190RhoGAP shRNAs on collagen gel contraction. Data are represented as mean $\pm$ standard error of the mean (SEM).

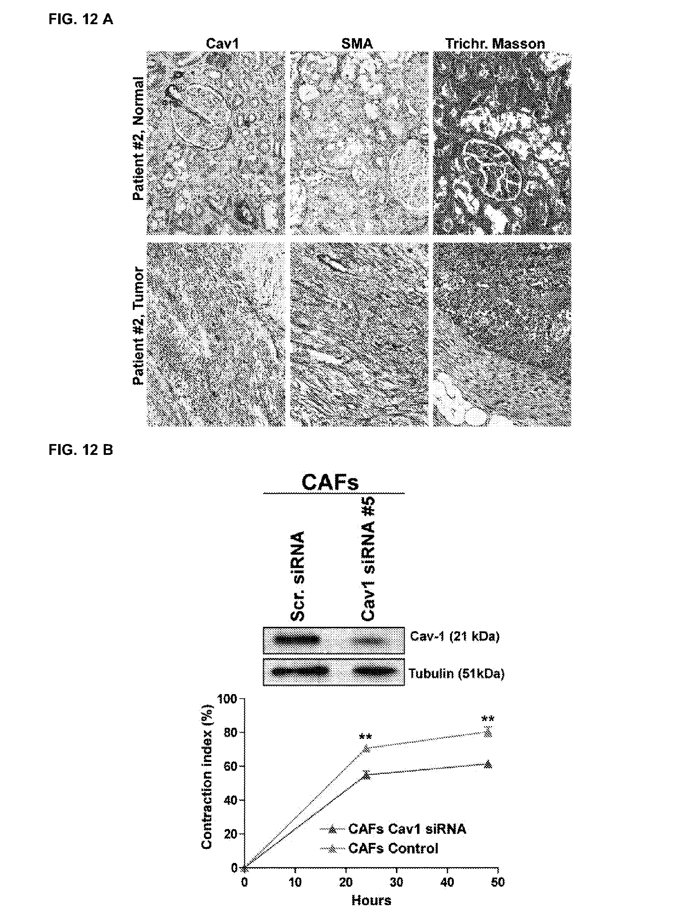

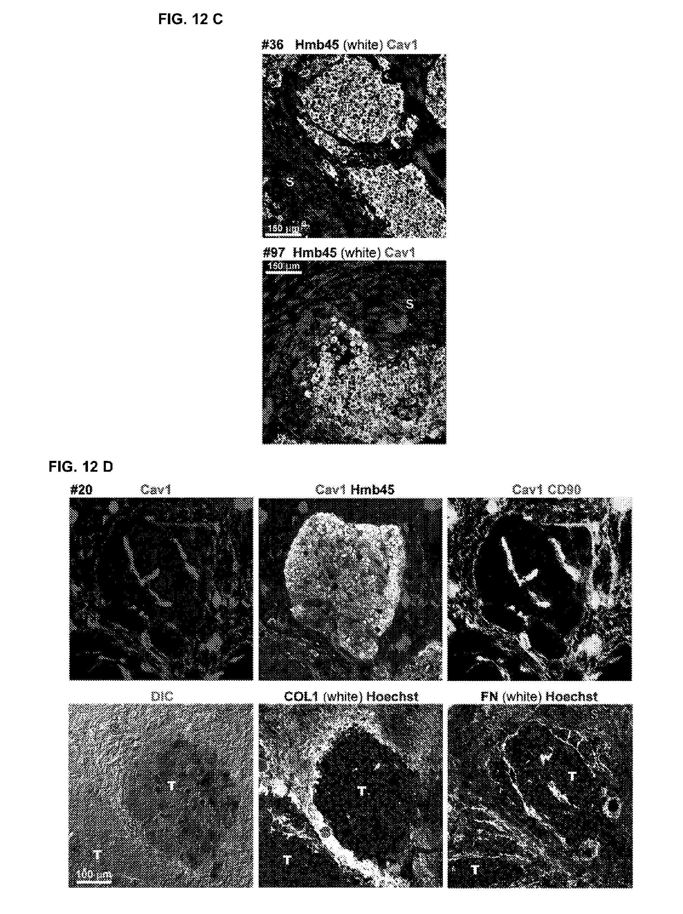

**Figura 12.** (A) Paired paraffin-embedded sections of normal kidney and renal tumor tissue from a representative patient. Sections are stained for Cav1, a-SMA, and Collagen deposition (Masson's trichrome) as indicated. (B) Fibroblasts from renal tumor tissue (CAFs) were isolated and infected with lentivirus encoding scrambled or Cav1-siRNA. Immunoblot shows Cav1 silencing efficiency. Tubulin shows equal loading. Collagen gel contraction assay confirms impaired contraction by Cav1-silenced CAFs. (C) Hmb45 (white) and Cav1 (red) staining in distant tumor-associated stroma of metastases from melanoma samples. (Note that in Patient #97 tumor cells are positive for Cav1.) (D) Staining for Cav1, Hmb45, CD90, Collagen (COL1), FN, and nuclei (Hoechst) in distant tumor-associated stroma of a melanoma metastasis. S = stroma, T = tumor. Data are represented as mean $\pm$ standard error of the mean (SEM).

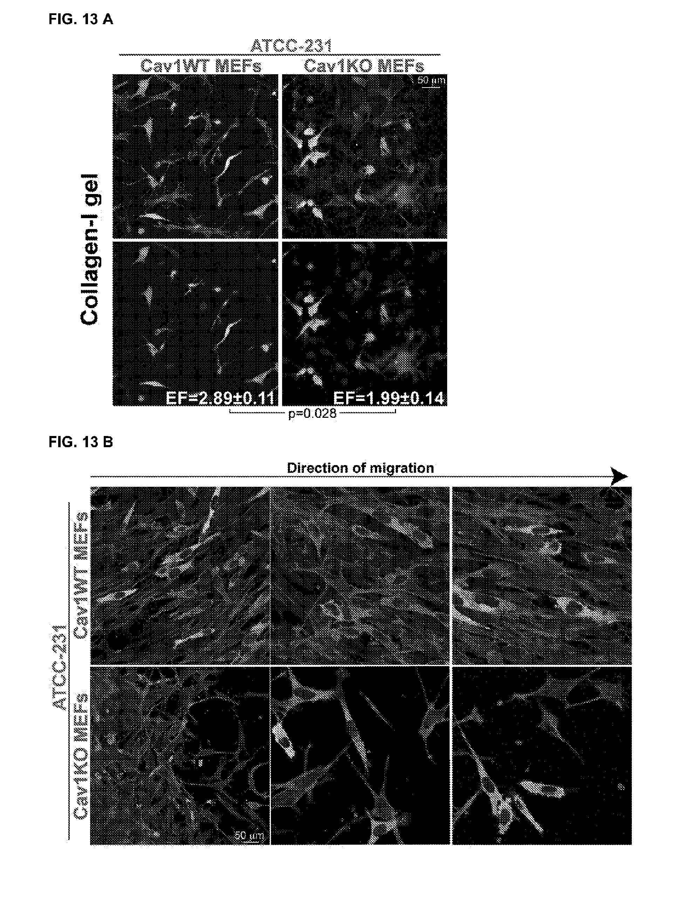

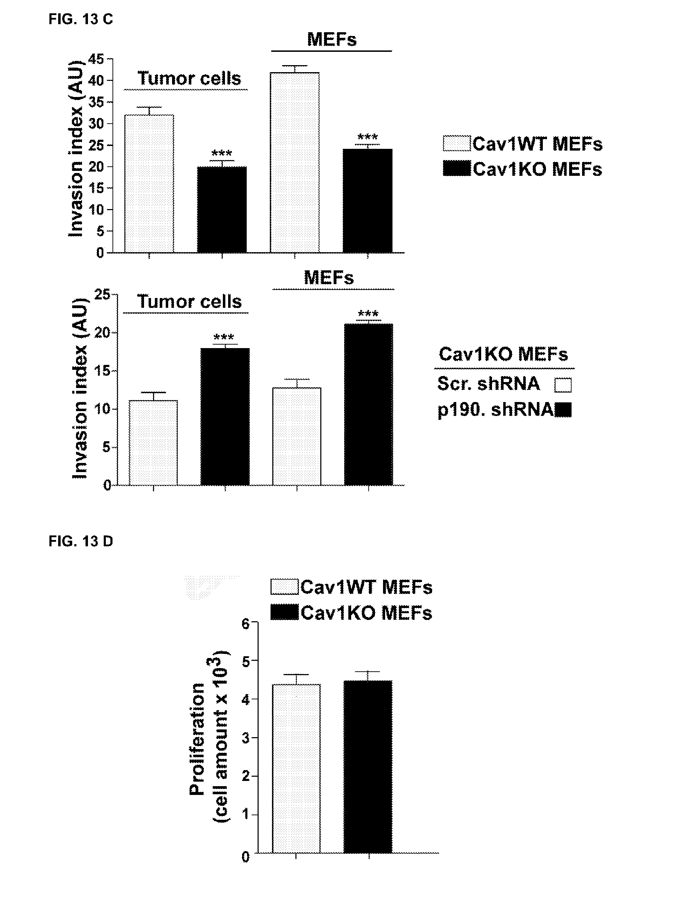

**Figura 13.** (A) A 1:1 mix of calcein-pre-labeled ATCC-231 cells and WT or Cav1KO MEFs was embedded in collagen gels, fixed, and stained for actin and nuclei (Hoechst). EFs of ATCC-231 cells are indicated. (B) Higher magnification of invading areas from Figure 6C, shown to highlight polarization and directionality of migration of ATCC-231 tumor cells and Cav1WTMEFs. (C) Invasion index of the experiment shown in Figure 6D calculated for tumor cells (PC3) and MEFs. (D) Quantification of MEF proliferation rate in the Matrigel invasion assay. (E) Tumor cells and MEFs were spatially separated during Matrigel invasion to mimic physiological topography. Start and end points are shown. The chart represents the invasion index for tumor cells (PC3) and MEFs. Data are represented as mean $\pm$ standard error of the mean (SEM).

**Figura 14.** (A) Orthotopic mammary gland allografts of E0771 tumor cells in WT or CavKO mice. (a) Primary tumor photon flux. (b) Quantification of the total number of metastatic foci and their organ distribution detected by bioluminescence *ex vivo.* (c) Quantification of SHG collagen content. (B) Orthotopic mammary gland xenografts of LM-4175 cells in WT or CavKO mice. (a) Multiphoton excitation microscopy of intact fixed tumors extracted from Cav1WT and KO mice 8 days after injection. Images show second harmonic generation (SHG) and GFP signal of LM-4175 cells. The same tumors were processed and stained with Masson's trichrome and picrosirius red (PR). Polarized light highlights fibrillar collagen. Arrows indicate cells with elongated proinvasive morphology in WT. S = stroma, T = tumor. (b) Quantification of the angle of collagen fibers angle to the tumor boundary (for SHG and PR). TACS = tumor-associated collagen signature. (c) Primary tumor photon flux in nude mice 102 days after xenografting. (C) Subcutaneous injection of Matrigel containing LM-4175 tumor cells plus the indicated pMEFs. (a) *In vivo* primary tumor photon flux. (b) Representative images of primary tumors stained for FN, SMA, and nuclei. (D) Experimental metastasis assay. (a) Experimental scheme. (b) Representative images of metastatic growth over time. (c) Plot of metastatic growth over time. Insets show representative images of photon flux in lungs at 20 days after allograft. Data are represented as mean $\pm$ standard error of the mean (SEM).

## DETAILED DESCRIPTION OF THE INVENTION

The present invention identifies Cav1 as a regulator of ECM remodeling and desmoplastic processes. CAFs, the main cell component of the desmoplastic stroma of solid cancers and their metastases, influence TC growth through paracrine secretion of growth factors and recruit endothelial progenitors to promote angiogenesis. CAFs deposit ECM and remodel the tumor stroma. ECM fiber tracks align at the tumor margin, and force- and protease-mediated ECM remodeling by fibroblasts favors TC invasion (Gaggioli et al., 2007 Nat. Cell Biol. 9, 1392-1400; Provenzano et al., 2008 Biophys. J. 95, 5374-5384). The inventors show that the stroma of breast, renal, and colon carcinoma and melanoma metastases is enriched in Cav1-expressing CAFs, and contractility of renal CAFs is reduced by lowering Cav1 levels. Cell-free 3D matrices generated by Cav1WT fibroblasts are stiffer and more aligned than matrices derived from Cav1KO fibroblast, thus better stimulating TC elongation, velocity, and directional migration. Orthotopic implantation in Cav1KO mice impairs tumor invasiveness and metastatic potency. In cocultures fibroblast Cav1 favors TC invasion, and coinjection of these cells into nude mice increases metastasis. Both effects depend on Cav1-regulated p190 activity. Results from the experimental metastasis assay show the important role of fibroblast Cav1 in events before intravasation. Thus CAF expression of Cav1 favors tumor progression via biomechanical remodeling of the primary tumor-associated stroma.

These findings may help explain the somewhat contentious role of Cav1 in tumorigenesis, as the protumorigenic action of Cav1 may depend on expression by stromal fibroblasts. The inventors have demonstrated that evaluation of tumorigenic potential requires examination of both tumor and stroma, especially for Cav1 expression. Cav1 is enriched in the stroma of various tumors, and stromal Cav1 is linked to poor survival.

The inventors have found a positive correlation between Cav1 and the CAF marker SMA

Previous studies proposed stromal Cav1 expression as a positive prognostic indicator in breast cancer, which is the opposite of the present invention. Moreover, these studies did not link Cav1 expression to CAFs or to a positive CAF marker, and loss of stromal Cav1 did not correlate with metastasis (Sloan et al., 2009 Am. J. Pathol. 174, 2035-2043;

Witkiewicz et al., 2009 Am. J. Pathol. 174, 2023-2034).

Cav1 has a complex role in tumor stroma, and the present invention a last provides a way to use of Cav1 for prognosis. The experimental results with orthotopic grafts and the stroma-dependent tumorigenicity assay show that increased fibroblast Cav1 promotes local invasiveness and metastasis via remodeling of the stromal ECM. Cav1 also regulates other (nonfibroblast) tumor-associated stromal components.

Through reciprocal cell-ECM interactions, the stroma provides environmental cues essential for tissue architecture. Cav1 loss in stromal cells produces benign stromal lesions causing abnormal epithelium growth and differentiation. The inventors have found that Cav1WT mammary glands show markedly higher SHG signal intensity and numbers of straight collagen fibers which favour TC migration, invasion and metastasis. The correlation between disorganized mammary stroma and impaired growth, invasion, and metastatic potency of orthotopic grafts shows that Cav1- positive stroma is permissive for tumor progression. Together, this shows that Cav1-dependent impaired architecture of native stroma affects tumor progression.

The FDMs generated in the present invention model the physiological microenvironment of cell interactions and reveal that the matrix generated by Cav1 fibroblasts increases cell elongation. Morphology of all cells tested was more strongly influenced by the surrounding 3D architectural organization than by its "intrinsic" shape in 2D. Cav1 phosphorylation at Tyr14 coordinates Rho GTPase activity (Grande-Garcia et al., 2007 J. Cell Biol. 177, 683-694).

The inventors show that Cav1 regulates p190 phosphorylation and partitioning into ordered domains, and that lack of Cav1 generates Rac accumulation in these sites. The inventors show that Cav1-expressing fibroblasts increase alignment of ECM fibers through Rho- and force-dependent matrix reorganization. In turn these matrices enhance cell elongation, mature integrin adhesions, and reduce cell protrusions. They also show that tumor cell elongation, velocity, and migration directionality are enhanced by Cav1 FDMs, as. coculture with Cav1 fibroblasts favors TC elongation and invasiveness *in vivo* and *in vitro*. Furthermore, the inventors have found that the tumor-stroma interface of mammary tumors in Cav1-expressing mice is more proinvasive than in their Cav1 KO counterparts, with increased collagen fiber alignment. The inventors have found a strong correlation between metastatic potency and intratumoral matrix alignment.

The results provided by the inventors identify a critical role for Cav1 in growth and invasion, highlighting a function of Cav1 in the regulation of matrix-dependent cell behavior.

The inventors show that Cav1 regulates Rho GTPase activity by modulating membrane partitioning of p190 and thereby its phosphorylation. Fibroblast expression of Cav1 *in vitro* and *in vivo* favors an organized 3D stromal architecture that promotes spindle morphology, facilitates TC invasion, and increases p190-dependent metastatic potency.

These findings correlate with increased numbers of Cav1-expressing CAFs in the stroma of human tumor samples. Cav1 silencing in human CAFs decreases their contractility, identifying a role for Cav1 in normal tissue homeostasis and pathological scenarios.

Therefore, a first aspect of the present invention relates to the use of *caveolin-1* in tumor-associated fibroblasts as biomarker of tumor progression.

Caveolin-1 is a protein that in humans is encoded by the Cav1 gene (*caveolin-1*). It is a scaffolding protein and it is the main component of the caveolae plasma membranes found in most cell types. Cav1 has been considered a tumor-suppressor gene, although its role is controversial.

The term tumor-associated fibroblasts (TAFs) and carcinoma-associated fibroblasts (CAFs) are used indistinctly in this description. TAFs can be identified *de visu* by specialized histologists when employing common histology stainings or can be stained with specific markers known in the art. The preferred TAF markers are smooth muscle actin (SMA), CD90 and vimentin, and SMA is the most preferred one. A list of TAF markers is provided by Dewever O et al (Int J Cancer. 2008, 15; 123(10):2229-38).

The expression "tumor progression" refers to tumor growth as well as to cell migration to produce both local invasion and distant metastasis.

The term "tumor" refers to a benign, pre-malignant or malignant neoplasm, including cancer.

Thus, in a preferred embodiment of the first aspect of the present invention, at least one further biomarker selected from: smooth muscle actin, CD90 and vimentin is used. Preferably, SMA is further used.

A preferred embodiment of the first aspect of the present invention is therefore the use of Cav1 and SMA as biomarker of tumor progression.

The results of the present invention show that intrinsic fibroblast Cav1 and the Cav1-regulated microenvironment cooperate to enhance cell elongation, migration, and invasion through force-dependent organization of the surrounding 3D environment. Cav1 mediates these effects by regulating Rho activity via changes in p190 localization and phosphorylation. These data show that this Cav1-dependent bidirectional cell/matrix mechanical crosstalk is critical for normal tissue homeostasis and architecture, and for tumor invasion and metastasis.

A second aspect of the present invention relates to a method for the diagnosis and/or prognosis of tumor progression comprising the following steps:

a) detection and/or quantification of an expression product of *caveolin-1* gene in TAFs of a sample isolated from a subject,

b) comparison of the expression levels of *caveolin-1* detected and/or quantified in step (a) with standard values,

c) finding a significant overexpression of *caveolin-1* in the comparison of step (b), and

d) attributing the significant overexpression of *caveolin-1* found in step (c) to a poor prognosis.

The expression "detection and/or quantification" of an expression product of Cav1 gene in TAFs of a sample isolated from a subject, refers to the detection of the presence and/or the measure of the quantity or the concentration of said expression product, preferably in a semiquantitative or quantitative manner. The measure can be direct or indirect. A direct measure refers to the measure of the quantity or the concentration of the Cav1 gene expression products in TAFs based on a signal that is directly related to the number of molecules of said expression products present in the TAFs of the sample. This signal can be measured by measuring the intensity of a physical or chemical property of the expression product. An indirect measure includes a measure obtained by using other components or a biological measuring system, such as measuring a cell response, a ligand or a tag or enzymatic reaction products.

The detection, as it is understood by a person skilled in the art, is not intended to be correct in a 100% of the analyzed samples. Nevertheless, it requires a statistically significant amount of the analyzed samples to be classified correctly. The amount that is statistically significant can be established by a person skilled in the art by means of different statistical tools, as for example, but not limited to, determining the confidence intervals, the p value, a Student's t test or a Mann- Whitney test or Fisher functions. Preferably, the confidence intervals are at least of 90% or at least of 95%, at least of 97%, at least of 98%, or at least of 99%. Preferably the p value is smaller than 0.1, than 0.05, than 0.01, than 0.005, or than 0.001. Preferably, the present invention allows the correct determination of the prognosis of at least 60%, at least 70%, at least 80% or in at least 90% of the subjects of a certain group or analyzed population.

The term "expression product" of Cav1 gene refers to either the messenger RNA or the protein generated when said gene is expressed. A person skilled in the art is aware of many methods that can be employed to detect and/or quantify such expression products, as for example, but not limited to, *in situ* hybridization, PCR, qPCR, northern blot, immunological methods such as immunohistochemistry, immunocytochemistry or western blot. A person skilled in the art knows how to design specific primers and or probes and/or antibodies against Cav1 gene expression products for their detection and/or quantification.

The term "sample" refers to an isolated biological sample that has been isolated from an organism as the human or animal body and comprises cells and physiological fluids. The biological sample can be a tissue, for example, but not limited to, a biopsy or a fine needle aspiration biopsy. In a preferred embodiment, the isolated sample of step (a) is a solid tissue. The biological sample can be, for example, but not limited to, fresh, frozen, fixed or paraffin-embedded.

The term "comparison" refers to the action of comparing Cav1 expression levels in the TAFs of the sample isolated from a subject that is supposed to have a tumor with Cav1 expression levels in the stroma of an isolated sample of a healthy or normal tissue, preferably with Cav1 expression levels of the fibroblasts of said healthy or normal tissue. The "standard values" are therefore the amount of Cav1 expression products in the stroma of a healthy or normal tissue, preferably in the stromal fibroblasts. For determining the standard values, a reference sample may be used. This reference sample may be analyzed simultaneously or consecutively with the sample isolated in step (a). The comparison of step (b) may be carried out manually or by computerized means.

The term "amount" refers to the absolute or relative quantity of Cav1 gene expression products.

The term "subject" refers to a vertebrate animal, preferably to a mammal, more preferably to a human. Mammals include, but are not limited to, farm animals, sport animals, pets, primates, and rodents.

The term "significant" refers to the statistically significant difference found in step (c) when comparing the levels of Cav1 expression products in the TAFs of the isolated sample with the levels of Cav1 expression products in the reference sample. A statistically significant different means that the p value is at least smaller than 0.5, smaller than 0.1, than 0.05, than 0.01, than 0.005, or than 0.001.

The term "overexpression" refers to the levels of Cav1 expression in the TAFs of the isolated sample of step (a) being higher than the levels of Cav1 expression in the reference sample.

The term "poor prognosis" refers to an increased progression of the tumor (including local invasion and distant metastasis), and to decreased patient survival.

The examples of the present invention show that Cav1 overexpression in TAFs correlates significantly with a smaller patient survival.

In a preferred embodiment of the second aspect of the invention, the step (a) further comprises the detection and/or quantification of the expression product of at least one of the following biomarkers: SMA, CD90 and vimentin. Preferably, step (a) comprises the detection and/or quantification of the expression product of SMA.

In a preferred embodiment of the second aspect of the invention, the expression product is an mRNA or a protein. The reference of the sequence of human Cav1 mRNA is GenBank accession number NM_001753, and the reference of the sequence of human Cav1 protein is GenBank accession number NP_001744.2.

In a preferred embodiment of the second aspect of the invention, the tumor is an epithelial tumor. Preferably, the tumor is of breast, kidney, colon or melanoma.

A third aspect of the present invention relates to a kit consisting essentially in specific primers and/or probes and/or antibodies for detecting and/or quantifying *caveolin-1* expression; and specific primers and/or probes and/or antibodies for detecting and/or quantifying the expression of at least one of the following genes: SMA, CD90 and vimentin, preferably of SMA.

In a preferred embodiment of the third aspect of the invention, the kit further comprises other reagents or materials as for example, but not limited to, buffers, complex forming proteins such as avidin, streptavidin and biotin, enzymes, labeled or unlabeled secondary antibodies, enzyme substrates, tubes, plates or any other additional materials needed to carry on any of the already described protocols for analyzing gene expression.

A fourth aspect of the present invention relates to a Caveolin-1 inhibitor for use as a medicament.

The term "inhibitor" refers to a substance or molecule capable of reducing or eliminating Cav1 protein activity, either by blocking its function or by reducing or eliminating its presence. The activity of Cav1 can indirectly be determined by a person skilled in the art using different methods already described in the art, as for example, but not limited to, measuring Rho GTPase activity by a Rho Pull down assay as described (Grande-Garcia et al., 2007 J. Cell Biol. 177, 683-694) and/or measuring cell-contractile ability of collagen gels as described in the present document. Direct determination of Cav1 can be determined by the quantification of Cav1 mRNA or protein.

In a preferred embodiment of the fourth aspect of the present invention the Caveolin-1 inhibitor further comprises an excipient. The term "excipient" refers to a component of a pharmaceutical composition or a medicament that is not an active compound but a diluent, a vehicle or a carrier, that is considered "pharmaceutically acceptable" when it is safe, non toxic and does not present adverse effects. The term "excipient" refers to a substance that helps the absorption of the compound, stabilizes it or helps in the preparation of the medicament to give it consistency or provide flavors that make it nicer. Thus, excipients may serve to maintain the ingredients together, such as starch, sugars o cellulose, to sweeten, to give color, to protect the medicament as for example to isolate it from air and/or humidity, to work as a filler in a pill, capsule or any other form of presentation as for example, dicalcium phosphate, to favor disintegration of the components and their absorption in the intestine, without excluding other types of excipients not mentioned in this paragraph.

In a preferred embodiment of the fourth aspect of the present invention, the medicament comprises a therapeutically effective amount of the Caveolin-1 inhibitor. The expression "therapeutically effective amount" refers to an amount that, administered in doses and during the necessary period of time, is effective in achieving the desired prophylactic or therapeutic effect. A "therapeutically effective amount" of the Caveolin-1 inhibitor of the invention may vary with the stage of the disease, the age, sex or weight of the subject, and refers to an amount that does not present adverse effects nor toxicity, and that us capable of achieving the desired prophylactic or therapeutic effect.

The Caveolin-1 inhibitors are preferably administered by parental administration, in the form of a sterile injectable aqueous or oleaginous suspension. The term "parenteral" as used herein refers to introduction into the body by way of an injection (i.e., administration by injection), including, for example, subcutaneously (i.e., an injection beneath the skin), intramuscularly (i.e., an injection into a muscle); intravenously (i.e., an injection into a vein), intrathecally (i.e., an injection into the space around the spinal cord), intrasternal injection, or infusion techniques. A parenterally administered composition of the described invention is delivered using a needle, e.g., a surgical needle. Injectable preparations, such as sterile injectable aqueous or oleaginous suspensions, may be formulated according to the known art using suitable dispersing or wetting agents and suspending agents. The administration of the medicament of the present invention is preferably administered locally, preferably by guided therapy directed to the tumor stroma.

In a preferred embodiment of the fourth aspect of the present invention, the medicament further comprises another active ingredient. The Caveolin-1 inhibitors may be combined with another active ingredient. An "active ingredient" is all matter of human, animal, vegetable, chemical o other origin whose activity is appropriate to constitute a medicament.

A fifth aspect of the present invention relates to a Caveolin-1 inhibitor for use in the prevention of tumor progression.

In a preferred embodiment of the fifth aspect of the present invention, the tumor is epithelial. Preferably, the tumor is of breast, kidney, colon or melanoma.

In a preferred embodiment of the fifth aspect of the present invention, the Caveolin-1 inhibitor is an interfering RNA or a blocking antibody. Preferably, the Caveolin-1 inhibitor is a small interfering RNA. More preferably, it is SEQ ID NO: 1 or SEQ ID NO: 2.

SEQ ID NO: 1 is the DNA sequence corresponding to a siRNA duplex corresponding to bases 301-319 from the open reading frame of the murine caveolin-1 mRNA. SEQ ID NO: 2 is the DNA sequence corresponding to a small interfering RNA (siRNA) corresponding to bases 254-277 of the human Cav1.

A small interfering RNA or short interfering RNA is a small double strained RNA molecule of 20-25 nucleotides that interferes with the translation of an mRNA, therefore inhibiting the production of the peptide or polypeptide codified by said mRNA, causing its deficiency. RNA interference can also be achieved by short or small hairpin RNAs that are delivered into cells by a vector and produce siRNAs inside the cell.

The term "antibody" refers to immunoglobulin molecules and immunologically active portions thereof, that is molecules that comprise a specific binding site in the antigen. There are five isotypes or main classes of immunoglobulins: IgM, IgD, IgG, IgA and IgE. A Caveolin-1 blocking antibody binds specifically to Caveolin-1 and inhibits its function and activity.

A sixth aspect of the present invention relates to p190RhoGAP (hereafter called p190) agonists for use as a medicament.

In a preferred embodiment of the sixth aspect of the present invention, the p190 agonists are p190 mRNA or p190 protein. Other p190 agonists can be determined by a skilled in the art by performing the following assay: a Rho Pull down assay to measure Rho Activity as described (Grande-Garcia et al., 2007 J. Cell Biol. 177, 683-694). The higher the activity of p190, the lower the activity or Rho. An alternative assay is to measure the cell-contractile ability of collagen gels as described in the present document. The higher activity of p190, the lower the ability of cells embedded in collagen gels to contract them.

There are several p190 agonists already described in the art, such as Rnd proteins Rnd1, Rnd2, and Rnd3 (RhoE) (Goh, L.L. and Ed, M. 2010 PLoS ONE, 5 (8), art. no. e1409; Wennerberg, K. et al. 2003 Current Biology, 13 (13) 1106-1115). Other p190 agonists can be selected from the ones described in US07915384.

A seventh aspect of the present invention relates to p190 agonist for use in the prevention of tumor progression. The inventors have shown that p190 promotes tumor encapsulation by controlling the reorganization of at least FN and collagen in the tumor stroma. Also, the inventors have shown a correlation between the alignment of this ECM components and the number of metastasis.

An eighth aspect of the present invention relates to Rho-A inhibitors for use in the prevention of tumor progression. In a preferred embodiment of the eighth aspect of the present invention, the Rho-A inhibitor is selected from the dominant negative form RhoN19, C3 toxin, an interfering RNA and a blocking antibody. The Rnd proteins mentioned above are also Rho-A inhibitors that can be used in the tumor stroma, preferably in the fibroblasts of the tumor stroma, to prevent tumor progression. Also, the Rho Kinase inhibitor Y-27632 may be used as Rho-A pathway inhibitor for use as a medicament and in the prevention of tumor progression. Rho Kinase is a direct Rho effector regulating this pathway downstream of Rho.

The administration of the medicaments of the present invention is preferably administered locally, preferably by guided therapy directed to the tumor stroma, preferably directed to the fibroblast of the tumor stroma, preferably to SMA expressing fibroblasts in the tumor stroma.

Throughout the description and the claims, the term "comprises" and its variants are not intended no exclude other technical characteristics, additives, components or steps. To a skilled in the art, other objects, advantages and characteristics of the present invention will emerge from the description and from the practice of the invention. The following examples and drawings are provided as an illustration and are not intended to limit the present invention.

EXAMPLES

Having described the invention in general terms, it will be more easily understood by reference to the following examples which are presented as an illustration and are not intended to limit the present invention.

Example 1:Cav1 Regulates Matrix-Induced Cell Morphology and Reciprocal Interaction with the 3D Microenvironment through Contraction

For a physiologically realistic culture substrate, we generated cell-free 3D matrices from confluent 8 day fibroblasts cultured in the presence of ascorbic acid; the resulting fibroblast-derived 3D matrices (FDMs) are rich in fibronectin (FN) and closely resemble *in vivo* mesenchymal matrices (Figure 1A, Figure 8A). We seeded FDMs with Cav1 wild-type (Cav1WT) and Cav1 knockout (Cav1KO) mouse embryonic fibroblasts (MEFs) and analyzed cell morphology. Growth in FDMs doubled the cell length: breadth ratio (elliptical factor/EF) of Cav1WT MEFs (Figure 1B and Figure 8B) and almost halved their surface area compared with growth on 2D FN (Figure 8C). In contrast, FDM culture only mildly affected the morphology of Cav1KO MEFs, though it increased the number of cell protrusions (Figures 1B and 1C). Similar results were obtained when cells were grown in collagen-I (Col-I) gels (Figure 1 D). Consistent with previously described Cav1-dependent Rac regulation (Grande-Garcia et al., 2007 J. Cell Biol. 177, 683-694), Cav1 deficiency increased PM targeting of Rac1 and its downstream effector phospho-S141-Pak1 (Figure 1E and Figure 8D). In contrast, phosphorylation of myosin light chain-2 (pMLC) was decreased, suggesting that Cav1 influences cell-induced matrix contractility. Consistently, Cav1WT MEFs contracted Col-I gels more effectively than Cav1KO MEFS at all cell concentrations tested (Figure 1F and Figure 8E). Re-expression of unmodified Cav1, but not its nonphosphorylatable mutant Cav1Y14F, rescued Rac1 localization, cell elongation, gel contraction, and pMLC levels in Cav1KO MEFs (Figure 1G). Cav1 thus regulates features of fibroblasts that influence mechanical 3D microenvironment remodeling.

Example 2: Cav1-Dependent Microenvironment Regulates Cell Shape, Protrusion Number, Rac1 Activity, and Morphology of Integrin-Dependent Adhesion Structures

The role of Cav1 in 3D microenvironment formation was tested. We compared culture in FDMs produced from immortalized Cav1WT and Cav1KO MEFs with 2D culture. Three-dimensional growth raised smooth muscle actin (SMA) expression preferentially in WT MEFs close to levels in primary cultures (pMEFs) (Figure 9A). When these FDMs were reseeded with Cav1WT or Cav1 KO MEFs, cells of both genotypes were more elongated when grown in FDMs generated by WT MEFs (Figure 2B and Figure 9B). These results show that lack of Cav1 alters FDM structure and composition; the most elongated cells were obtained when Cav1WT MEFs were plated in Cav1WT FDMs. Re-expression of Cav1 in Cav1KO MEFs rescued the ability to generate pro-elongation 3D matrices, whereas re-expression of Cav1Y14F had no effect (Figures 2C and 2D). Thus Cav1, through residue Tyr14, favors fibroblast elongation directly through endogenous expression and indirectly through cell dependent 3D microenvironment remodeling, indicating that endogenous Cav1 and the Cav1-dependent microenvironment cooperate to enhance cell polarity. Cav1WT FDMs also reduced the number of cell protrusions (Figure 2E and Figure 9C) and Rac-GTPase activity (Figure 2F) independently of Cav1 expression by seeded cells, confirming the ability of Cav1-dependent ECM to favor *in vivo*-like spindle morphology. We also assessed the impact of the Cav1-dependent microenvironment on the formation of 3D-matrix adhesions. Integrin dependent 3D-matrix adhesions differ from regular FA in molecular composition (lower pY397FAK levels) and are longer (up to 19 mm) and thinner. Cav1WT and Cav1KO FDMs both decreased adhesion-targeted pY397FAK levels compared to a 2D FN substrate (Figure 9D). The longest 3D-matrix adhesions were obtained when Cav1WT MEFs were plated in Cav1WT FDMs (11.14 $\pm$ 0.48 mm) (Figure 2G and Figure 9E), indicating that both matrix and cells are important for determining adhesion length. The matrix adhesion marker vinculin localizes to 3D-matrix adhesions and its recruitment is force dependent, and the mobile vinculin fraction, determined by fluorescence recovery after photobleaching (FRAP), is an index of adhesion-dependent force increase. Cav1KO FDMs increased the mobile vinculin fraction (Figures 2Ha-2Hc), indicating that these matrices slow the maturation of adhesion structures and the generation of adhesion-dependent forces. Endogenous Cav1 expression contributes little to the maturation of 3D-matrix adhesions (Figures 2Hb and 2Hc), contrasting the strong influence of the assorted FDMs.

Example 3: Cav1 Promotes Patterning and Stiffness of 3D Matrices thus Regulating Normal Tissue Architecture

To assess the impact of Cav1 expression on microenvironment organization, compliance, and composition, we measured FN fiber orientation in FDMs. FN fibers in Cav1WT-derived FDMs were more parallel than in Cav1KO FDMs (Figures 3A and 3B). The level of Cav1 WT FDM organization was consistent with published results for normal fibroblasts. A similar level of parallelism was obtained in FDMs generated by Cav1 KO MEFs re-expressing Cav1WT, but not Cav1Y14F (Figures 3C and 3D). Therefore, Cav1, via Tyr14, plays an important role in the topographical organization of 3D microenvironments. Higher-than-normal parallelism is characteristic of tumor stromal collagen fibers and matrices generated by CAFs (Provenzano et al., 2008 Biophys. J. 95, 5374-5384). Moreover, a stiff and ordered 3D microenvironment is essential for TC invasiveness, and highly aligned and organized collagen fibers increase matrix strength and stiffness *in vivo* (Gaggioli et al., 2007 Nat. Cell Biol. 9, 1392-1400; Provenzano et al., 2008 Biophys. J. 95, 5374-5384)). Evaluation of FDM stiffness by atomic force microscopy (AFM) revealed that the Young Modulus of Cav1WT-derived FDM is 40% larger and has a wider standard deviation than that of Cav1KO FDM (Figure 3E and Figure 10A). Lack of Cav1 in MEFs thus makes the self-derived ECM less stiff and unifies its mechanical response. Cav1WT- and Cav1KO-derived FDMs were of equal thickness, and there were no significant differences in cell density before extraction (Figure 10B). Proteomic analysis revealed only slight, nonsignificant differences in the relative abundance of ECM proteins in WT and Cav1KO FDMs (Figure 10C and table 1).

Table 1. Identified proteins and relative ratios.

| Identified Proteins | Mouse IPI* Accession Number | Method A Ratio 115/114 (Unique peptides) | Method B Ratio 116/117 (Unique peptides) |
|---|---|---|---|
| Fibronectin 1 | gi\|46849812 | 1,06 (8) | 1.13 (4) |
| Perlecan (HSPG) | gi\|94373894 | 1.4 (9) | 1.01 (3) |
| Procollagen, type I, alpha 2 | gi\|6680980 | 0.63 (3) | 0.75 (3) |
| Fibrillin 1 | gi\|6679759 | 0.81 (3) | 0.82 (1) |
| Procollagen, type I, alpha 1 | gi\|34328108 | 0.71 (2) | 0.66 (2) |
| Fibulin 2 | gi\|6679757 | 1.2 (1) | N.D |
| Procollagen, type VI, alpha 3 | gi\|94363816 | 0.82 (1) | N.D |

(continued)

| Identified Proteins | Mouse IPI* Accession Number | Method A Ratio 115/114 (Unique peptides) | Method B Ratio 116/117 (Unique peptides) |
|---|---|---|---|
| Elastin microfibril interfacer 1 | gi|19527130 | N.D | 1.06 (1) |
| Chondroitin sulfate proteoglycan 4 | gi|20467423 | N.D | 0.81 (1) |
| Procollagen, type III, alpha 1 | gi|33859526 | N.D | 0.85 (1) |
| Nebulin | gi94368214 | 0.73 (1) | N.D |
| Otoferlin isoform a | gi94398959 | 0.72 (1) | N.D |
| Transglutaminase 2, C polypeptide | gi6678329 | 0.82 (2) | N.D |
| Reelin | gi6755312 | N.D | 1.02 (1) |
| Serine/arginine repetitive matrix 2 | gi30424862 | N.D | 0.63 (1) |

* International Protein Index of the EBI (European Bioinformatics Institute).

Examination of tissue architecture in Cav1KO mice by Masson's trichrome and picrosirius red (PR) staining revealed a thicker and less organized collagen-rich dermis in both adult and young mice (Figure 3F and Figure 10D). We next used multiphoton excitation microscopy coupled to second harmomic generation (MPE-SHG) imaging to study tissue organization at the mammary gland epithelial-stroma interface. SHG signal intensity was higher in Cav1WT mammary glands, in both the gland vicinity (Figure 3G) and neighboring stroma (Figure 10E), indicating greater collagen fiber organization. Tissues also contained collagen fibers devoid of SHG signal, which were often kinked in Cav1KO mammary glands but almost all straight in Cav1WT (Figure 3Ga and Figure 10E). PR staining confirmed disturbed collagen fiber organization in Cav1KO mammary glands, highlighting deficiency in fibrillar collagen around the acinus (Figure 3Gb).

Example 4: Cav1 Promotes Force-Dependent Remodeling of the Surrounding Environment via Rho GTPase Activation

FN fibrillogenesis relies on Rho GTPase-dependent cell contractility, which generates tensile forces that expose cryptic selfassembly sites in stretched FN molecules. Although their fibrillogenesis was delayed, Cav1KO MEFs were able to remodel the FN coating at later time points (Figure 11A) with similar FN expression levels as Cav1WT MEFs (Figure 11B). Absence of Cav1 thus delays but does not negate FN remodeling. Re-expression of Cav1 in Cav1KO MEFs restored cell-mediated FN fibrillogenesis (Figure 4A); re-expression of Cav1Y14F had no effect (Figure 11C). Cav1 deficiency decreases Rho activity via the endogenous inhibitor p190 (Grande-Garcia et al., 2007 J. Cell Biol. 177, 683-694). Constitutive activation of Rho GTPase in Cav1KO MEFs rescued the FN remodeling phenotype but had no effect on Cav1WT MEFs (Figure 4A). Defective FN remodeling in Cav1KO MEFs was also reversed by stable silencing of p190 (Figure 4B and Figure 11 Da), which decreased Rac activity and PM localization (Figure 4C and Figure 11Db). p190 knockdown (KD) also increased pMLC levels (not shown) and rescued cell contractility (Figure 4D). Moreover, p190 KD in Cav1KO MEFs increased the parallel fiber organization of derived matrix (Figure 4E), and this matrix increased the EF and decreased the number of protrusions of seeded Cav1WT MEFs (Figure 4F). Off-target effects were excluded with additional shRNAs (Figures 11E and 11F).
p190 activation is associated with its phosphorylation and partitioning into PM-ordered domains, where it inhibits Rho activity. We reported mildly increased p190 expression and phosphorylation in the absence of Cav1 (Grande-Garcia et al., 2007 J. Cell Biol. 177, 683-694). In contrast to Rac1 and pPAK, p190 PM targeting was independent of Cav1 expression (Figure 4G). However, p190 phosphorylation at the PM was higher in the absence of Cav1 (Figure 4H). Cav1 absence also promoted p190 partitioning into PM-ordered domains, an effect reversed by Cav1 re-expression (Figure 4I). Cav1 thus appears to remodel the 3D microenvironment by stimulating Rho-dependent cell contractility and to regulate Rho activity by controlling the phosphorylation and partitioning of p190 into membrane-ordered domains.

Example 5: Stroma of Human Breast, Kidney, and Colon Carcinoma and Melanoma Metastases Are Enriched in Cav1-Expressina Fibroblasts

We analyzed tumor-associated stroma of various carcinomas. Because Cav1 expression modifies mammary tissue architecture, we immunostained for Cav1 and SMA in a tissue microarray of 132 primary breast tumors and 35 normal breast tissues, scoring specifically for expression level in fibroblastic stroma (Figures 5A-5C and Tables 2 and 3). Expression levels of Cav1 and SMA were significantly higher in tumor stroma (p < 0.0001) and showed a mild statistical correlation (r = 0.54) (Figure 5B). Mean and median SMA values were marginally higher in high-than in low-stage disease (Wilcoxon p = 0.064). Moreover, Cox proportional hazards analysis showed that patients with stromal Cav1 expression had a 2.5 times higher 10 year mortality risk (Figures 5C and 5D). *In nitro* assessments showed that Cav1 expression was significantly higher in breast CAFs than in normal fibroblasts (NFs) (Figure 5E). Stromal Cav1 expression is thus a potential indicator of breast carcinoma progression. Tissue sections from five patients with renal carcinoma showed high tumor stromal Cav1 expression localized to blood vessels and CAFs (Figure 5F and Figures 12A and table 4). In contrast, Cav1 expression was low in healthy tissue and was mostly restricted to blood vessels and glomerular tubules. Stromal Cav1 expression in tumor sections was accompanied by a clear stromal reaction shown by positive SMA staining and collagen deposition (Figure 5F and Figure 12A). Next, NFs and CAFs harvested from patient #2 (Cav1 homogeneously stained the tumor-associated stroma) were induced to secrete and organize their own 3D *in vivo*-like matrices. Three-dimensional CAF cultures showed strong Cav1 and characteristic myofibroblast SMA expression (Figure 5G). We tested the effect of Cav1 KD on the ability of CAFs to contract Col-I gels. Similar infection efficiencies were obtained, but Cav1 KD was more efficient in NFs (Figure 5H). CAFs retracted Col-I gels more efficiently than NFs (Figure 5I). Mild Cav1 KD in CAFs was sufficient to decrease contraction to NF levels (Figure 5I). Similar results were obtained with a different Cav1 siRNA (Figure 12B). Cav1 thus contributes to the contractile phenotype of CAFs, and its stromal expression indicates renal carcinoma progression.

Table 2. Descriptive statistical representations in the patient cohort (N = 140).

|  | *N* |  |
|---|---|---|
| Age (years) | 120 | 58.58 $\pm$ 13.04 (median = 56) |
| Race | 139 |  |
| White |  | 89.7% (105) |
| Black |  | 9.4% (11) |
| Other |  | 0.9% (1) |
| AJCC stage | 137 |  |
| 1 |  | 29.5% (41) |
| 2a |  | 16.5% (23) |
| 2b |  | 36.7% (51) |
| 2x |  | 12.2% (17) |
| 3a |  | 2.2% (3) |
| 3b |  | 0.7% (1) |
| 3c |  | 0.7% (1) |
| Grade | 106 |  |
| 1. Well-differentiated |  | 0.9% (1) |
| 2. Moderately-differentiated |  | 28.3% (30) |
| 3. Poorly-differentiated |  | 70.8% (75) |
| ER status Negative Positive | 124 | 35.5% (44) 64.5% (80) |
| HER2 status | 87 |  |
| 0 |  | 44.8% (39) |
| 1 |  | 25.3% (22) |
| 2 |  | 12.6% (11) |
| 3 |  | 17.2% (15) |

Table 3. Multivariate survival results for Cav1-positive and -negative stroma.

| | Stromal Cav1 | |
| --- | --- | --- |
| | ** | * |
| Hazard ratio | 10 years | overall |
| Ratio | 0.3766 | 0.4136 |
| 95%CI of ratio | 0.1983 to 0.7151 | 0.2251 to 0.7601 |
| p value (Wilcoxon test) | 0.0096 | 0.0388 |

Table 4. Scoring of stromal activation and Cav1 expression in normal kidney and renal tumor tissue from five patients.

| Patient number | Normal vs Tumoral | Cav1 levels | Description | Carcinoma type | Stage | Grade |
| --- | --- | --- | --- | --- | --- | --- |
| #1 | Normal | | Blood vessels only, glomerular capsule | Sarcomatoid in RCC + Papillary | T3bN2M0 | High |
| | Prim. tumor | + | Stromal cells | | | |
| #2* | Normal | - | Blood vessels and distal tubules (epithelium) | Clear cell carcinoma | T3NxM1 | 4 |
| | Prim. tumor | ++ | Stromal cells | | | |
| #3 | Normal | - | Blood vessels only, glomerular capsule, tubules | Sarcomatoid in RCC | T3aN2M1 | High |
| | Prim. tumor | ++ | Stromal cells | + Papillary | | |
| #4 | Normal | - | Blood vessels and marginal tubules (epithelium) | Clear cell carcinoma | T1bN0Mo | 2 |
| | Prim. tumor | + | Stromal cells | | | |
| #5 | Normal | - | Backround, blood vessels | Clear cell carcinoma | T3bN2M1 | 4 |
| | Prim. tumor | + | Stromal cells | | | |

*Extracted fibroblasts.

We next explored fibroblast Cav1 expression in colorectal cancer stroma, as this is one of the most common causes of cancer death in Western countries and its outcome is determined by the occurrence of metastatic dissemination. In normal tissue, Cav1 is expressed in smooth muscle cells of the muscularis mucosae, in blood vessels, and to a lower extent in the stroma underlying the epithelium (Figure 5J). Analysis of a tissue microarray of 84 colorectal carcinomas detected Cav1 expression in tumor-associated stroma but not in the epithelial TCs (approx. 98% of cases). Cav1 staining intensity scores revealed strong expression in 75% of tumor-associated stroma (Figure 5J). Cav1 was also expressed in the peritumoral stroma of all metastatic foci examined in a set of human melanoma metastatic explants, with >80% of fibroblasts Cav1 positive (Figure 5K). Cav1 was found in 79% of fibroblasts expressing SMA and 82% expressing CD90, a marker of human CAFs (Figure 5K). Cav1 expression was detected in very few metastatic (Hmb- 45+) TCs and in only 36% of infiltrated (CD45+) leukocytes (Figure 5K and Figure 12C). Cav1-expressing CAFs were excluded from tumor nests but were accompanied by heavy collagen and light FN deposits in the immediate peritumoral stroma (Figure 12D). Fibroblasts positive for CD90 and Cav1 were detected in distant tumor-associated stroma but were absent from normal connective or muscle tissue. Cav1 in nontumor tissue was localized exclusively in blood vessels. Stroma of metastatic foci is thus enriched in Cav1-positive CAFs, showing that stromal Cav1 is instrumental in both primary and secondary tumor niches.

Example 6: Cav1-Dependent 3D Microenvironment Stimulates *In Vitro* Tumor Cell Migration and Invasion

To investigate the mechanism by which microenvironment remodeling by stromal Cav1 affects TC behavior, we used three metastatic cell lines as representative models: MDA-MB-231 parental breast cancer cells (here called ATCC-231) and derived lines selected for *in vivo* tropism to lung (LM-4175) and bone (BM-1833). All three lines express high levels of Cav1. Metastatic cells seeded in FDMs generated by Cav1WT MEFs had a higher EF than when seeded in Cav1KO FDMs (Figure 6A). Cav1WT matrices also supported higher directionality and velocity of migration by TCs (Figure 6B). Although coculture with Cav1WT MEFs in Col-I gels only slightly increased TC EF (Figure 13A), it clearly favored TC invasion of the surrounding gel (Figure 6C and Figure 13C). Invading Cav1WT fibroblasts and TCs were both highly polarized and oriented perpendicular to the "tumor boundary" (Figure 13B). In an alternative matrigel-based 3D invasion assay, Cav1WT MEFs similarly promoted invasion by PC3 prostate TCs without displaying any differences in proliferation rate (Figure 6D and Figures 13C-13E), showing that fibroblast Cav1 is a general promoter of TC invasion. In both models Cav1WT MEFs invaded significantly faster than Cav1KO MEFs and preceded TCs. Similar results were obtained when fibroblasts were spatially separated from TCs by a thin matrigel layer in order to mimic tumor-stroma topography (Figure 13E). p190 KD in Cav1KO MEFs restored Cav1WT fibroblast-induced tumor cell invasiveness (Figure 6D and Figure 13C).

Example 7: Cav1-Dependent 3D Microenvironment Stimulates *In Vivo* Tumor Cell Invasion and Increases Metastatic Potency

The contribution of fibroblastic Cav1 to stroma-dependent tumorigenesis *in vivo* was explored in a luminescence-based orthotopic mammary gland tumor formation assay, in which luminescent and GFP-expressing cells from mammary adenocarcinoma E0771 were injected into mammary glands of syngenic WT and Cav1 KO mice (Figure 7A).

Absence of host Cav1 slightly decreased primary mammary tumor growth (Figure 7Ab and Figure 14Aa) and metastasis formation (Figure 14Ab). No significant differences were observed when cells were injected in tail vein; in fact Cav1KOs appeared to have a slight advantage over WTs (Figure 14D). We next studied tissue architecture at the tumor-stroma interface by MPE-SHG. Tumors grown in Cav1KO mice were minimally invasive: TCs were localized in regions surrounded by few kinked and disorganized collagen fibers (Figures 7Ac and 7Ad). In contrast, tumors grown in Cav1WT mice were collagen-rich, with invading TCs in intimate contact with aligned straight and curly collagen fibers (Figures 7Ab and 7Ac). Consistent with proinvasion radial alignment of collagen fibers relative to tumor boundary, the angle of collagen fibers was significantly more perpendicular in WT than in Cav1KO hosts (Figure 7Ad), showing higher invasivity in WT environment.

Table 5. Quantification of primary tumors stained for FN, SMA, and nuclei. The angles of single thresholded FN-labeled fibers were calculated and the percent of fibers within $\pm 20°$ compared. EF and angle distribution of SMA positive cells is shown.

| | % of fibers within $\pm 20°$ | SMA +cells EF | % of SMA+cells Within $\pm 20°$ |
|---|---|---|---|
| 'Cav1WT' tumors | 42.8±1.8% (n=5) | 2.08±0.08 (700) | 53.0±4% |
| 'Cav1KO' tumors | 28.8±2.6% (n=5) | 1.62±0.04 (933) | 32.1±3% |

To rule out effects derived from tumor angiogenesis, vessel leakiness, and extravasation, we conducted the following experiments. Orthotopic mammary gland tumors were obtained by injecting LM475 human TCs into lethally irradiated Cav1WT or Cav1KO mice (Figure 7B). After 9 days, equal-size tumors were either processed or transplanted together with their surrounding stroma into nude mice (Figure 7Ba). In this system, Cav1-dependent stroma is the only variable likely to affect TC invasiveness and metastatic potency. Using MPE-SHG imaging and histology, we evaluated Cav1-dependent matrix remodeling at the tumor-stroma interface of early tumors (8 days). Tumors grown in Cav1KO mice were minimally invasive. Collagen fibers were stretched and distributed tangentially along the tumor boundary, constraining tumor growth and consistent with previously described TACS-2 (Tumor-associated collagen signature-2) (Figures 14Ba and 14Bb). Indeed, tumors generated in Cav1KO mammary glands grew slower before and after transplantation into nude mice (Figure 14Bc). In contrast, WT tumors had an invasive morphology, with both TCs and collagen fibers aligned in the direction of invasion (approx. 90° to the tumor boundary), typical of TACS-3. After transplant, such tumors generated significantly more metastases in most organs analyzed (Figure 7Bb). These tumors moreover displayed significantly increased matrix fiber parallelism and alignment, and elongation of SMA-positive CAFs (Figures 7Bc and 7Bd and table 5). Interestingly, alignment of intratumoral FN fibers correlated with the number of metastasis (n = 10, Figure 7Bd). These results indicate that Cav1-positive surrounding stroma regulates primary tumor growth and favors invasion and metastasis. Because Cav1 was absent in all tissues, neither of the approaches described tests the effect of specific lack of Cav1 in the tumoral stroma associated with the orthotopic xenograft. To overcome this and to test the role of p190 in metastatic potency *in vivo,* we designed a luminescence-based subcutaneous tumor formation assay in which Matrigel-embedded luminescent TCs (LM-4175) were mixed with pMEFs (Cav1WT, Cav1KO MEFs, or p190-silenced Cav1KO) (Figure 7Ca) and injected subcutaneously into nude nice (Figure 7C). The microenvironment-remodeling properties of pMEFs are similar to those of their immortalized counterparts. p190 was stably silenced in Cav1 KO pMEFs and Cav1 was expressed only in Cav1WT pMEFs (Figure 7Ca).

Table 6. Quantification of primary tumors stained for FN, SMA, and nuclei. The angles of single thresholded FN-labeled fibers were calculated and the percent of fibers within ± 20° compared. EF and angle distribution of SMA positive cells is shown.

| | % of FN fibers within ±20° (number of tumors) | SMA + Cells EF (number of cells) | % of SMA+cells within ±20° |
|---|---|---|---|
| LM-4175 alone | 36.8±0.7%(n=5) | 1.70±0.03 (224) | 33.1±4% |
| LM-4175 + Cav 1WT MEFs | 50.3±2.3% (n=8) | 2.14±0.03 (1246) | 51.3±2% |
| LM-4175 + Cav 1 ko MEFs | 41.5±1.1% (n=10 | 1.68±0.02 (763) | 35.3±2% |
| LM-4175 + p190sh KO MEFs | 53.5±2.9% (n=7) | 2.08±0.03 (945) | 53.9±2% |

There was no difference in primary tumor growth (Figure 14Ca and table 6); but Cav1WT pMEFs were more potent stimulators of metastasis formation than Cav1KO pMEFs (Figures 7Cb and 7Cc), confirming the results obtained in orthotopic mammary assays and validating this assay for studying the mechanism of stroma-dependent tumor invasion and metastasis. Deficiency in metastasis deposition was rescued by p190 KD in Cav1KO pMEFs (Figures 7Cb-7Cd). Study of intratumoral stroma remodeling in tumor explants revealed an increase in FN fiber alignment when injected pMEFs expressed Cav1. Deficient alignment was rescued by p190 KD in Cav1KO MEFs (Figure 7Cc and Figures 14Cb and 14Cc). Intratumoral FN fiber alignment and metastatic potency were statistically correlated (n = 30, Figure 7Cd). Additionally, rescue of normal Rho activity (in p190-silenced Cav1KO pMEFs) increased alignment and elongation of SMA-positive fibroblasts (Figure 14Cb). Together these results demonstrate that stromal Cav1, through p190-dependent regulation of Rho-mediated cell contractility, remodels peri- and intratumoral microenvironments to facilitate TC invasion and metastasis. These data identify an important role for stromal-expressed Cav1 in tumor progression, consistent with the human tumor histology data, and highlight the importance of mechanical remodeling of the microenvironment in tumor metastatic potency.

## EXPERIMENTAL PROCEDURES

### Antibodies and Reagents

Ascorbic acid, ammonium hydroxide, and Triton X-100 were from Sigma. Primary antibodies: Rac and p190RhoGAP (Upstate Biotechnology); Rho- GDI (Santa Cruz), Cav1, CD90, GM130, CD45, and activated b1-integrin (9EG7) (BD); g-tubulin, SMA, FN, and Col-I (Sigma); Hmb45 (Dako), pY397FAK, and PAK1/2/3[pS141] (Biosource); GADPH and 4G10 (Millipore). Secondary antibodies were from Molecular Probes and from Jackson. FN was purified from human plasma.

**Cell Culture**

pMEFs and 3T3-immortalized MEFs from WT and Cav1KO mice were cultured as described (Cerezo et al., 2009 Mol. Cell. Biol. 29, 5046-5059). ATCC-231, LM-4175, and BM-1833 were cultured as described (Minn et al., 2005 Nature 436, 518-524). PC3 and E0771 TCs were cultured as recommended.

**Mice**

Cav1-deficient mice (STOCK Cav1tm1Mls/J) and WT B6129SF2/J controls were from The Jackson Laboratory (USA). Cav1-deficient mice and WT littermates (B6.Cg-CAV1tm1mls/J) were used for *in vivo* experiments. Female athymic nude mice were obtained from Harlan.

**3D Cell Culture and Assays**

Extracellular Matrices Produced by Immortalized or Primary Fibroblasts

Three-dimensional matrices reminiscent of *in vivo* ECM were prepared. Briefly, $2.5 \times 10^5$ cells/ml were plated on chemically crosslinked gelatin on tissue culture dishes or coverslips and maintained in confluence for 6-8 days. Cells were supplemented every 48 hr with 50 mg/ml fresh L-ascorbic acid (AA) to stabilize ECM components thus facilitate collagen production and polymerization. The resulting 3D cultures were checked for quality by indirect immunofluorescence or cells were removed from the matrix by alkaline detergent extraction, yielding cell-free 3D matrices for further analysis.

Collagen Gel Contraction Assay

$1.2 \times 10^5$ fibroblasts (alternatively, $6 \times 10^4$ CAFs/NFs) were mixed with NaOH-titrated collagen I (PureCol, INAMED) to a final collagen I concentration of 1 mg/ml. In some experiments, $4 \times 10^5$, $1 \times 10^6$, or $1.5 \times 10^6$ fibroblasts were used (Figure 8). The mixture was immediately transferred to a 24-well plate and lattices were allowed to solidify for 20 min at room temperature. Serum-containing medium was added to each well and gels were manually detached by circular movements using a sterile pipette tip. Gels were placed at 37°C and contraction was documented. Four or five fibroblast-containing gels were assayed for each condition. Gel contraction index was calculated with Metamorph software from the gel surface area measured on acquired images, and reported as the percentage of contraction of the initial surface area.

Spheroid Invasion Assay

The protocol is based on (Gaggioli et al., 2007 Nat. Cell Biol. 9, 1392-1400). ATCC-231 tumor cells were labeled (30 min) with calcein and mixed 1:1 with MEFs. The mixes were embedded in serum-free collagen I gels (15 ml, 1 mg/ml) in the well centers of an 8-well IBIDI chamber. After polymerization, the gel plug containing the cells was embedded in a second serum-containing collagen gel (150 ml, 1 mg/ml) and covered with serum-containing medium supplemented with SDF-1 a (50 ng/ml). After 6 days, gels were fixed, labeled with fluorescently conjugated phalloidin, imaged, and analyzed. Invasiveness was quantified as the area invaded by tumor cells.

Matrigel Invasion Assay

Equal numbers of GFP-expressing PC-3 cells and fibroblasts were mixed, loaded into the well centers of Matrigel-coated IBIDI angiogenesis wells, and covered with a 1:1 mix of Matrigel and medium (11 ml). After polymerization, chambers were filled with medium containing 2% FBS. Alternatively, PC-3 cells and fibroblasts were separated by a thin Matrigel layer. For this, PC-3 cells were first seeded into Matrigel and a thin Matrigel layer was overlaid three hours later and left to polymerize before seeding MEFs. During the 6 day invasion period, 5 ml medium containing 10% serum was added to the chambers every second day. The cells were fixed and labeled with phalloidin and Hoechst, and imaged by confocal microscopy. Invasiveness of each cell type was quantified as the area invaded. Total numbers of MEFs at the end point were quantified by automated counting of nuclei in all the acquired planes (ImageJ) and subtraction of GFP-positive (PC3) cells.

The spheroid invasion assay was adapted from Gaggioli et al. (2007) (Nat. Cell Biol. 9, 1392-1400) After 6 days, invaded gels were fixed, labeled with fluorescently conjugated phalloidin, imaged, and analyzed. Invasiveness was quantified as the area invaded by tumor cells. Col-I gel contraction assays were performed as published. Four or five fibroblast-containing gels were assayed for each condition. Gel contraction index was calculated with Metamorph software from the gel surface area measured on acquired images and reported as the percentage of contraction of the initial surface area.

## Tumorigenicity Assays and Bioluminescent Imaging

Orthotopic Xenografts, Subcutaneous Tumorigenicity Assay, Experimental Metastasis Assay, and Bioluminescent Imaging

In the first orthotopic xenograft protocol (Figure 7A), E0771 C57BL/6 mammary adenocarcinoma cells were lentivirally infected a vector encoding GFP and luciferase. GFP-positive cells (4 3 106 in 200 ml PBS:Matrigel 1:1) were injected into mammary glands of WT and Cav1KO mice (strain B6.Cg-CAV1tm1mls/J). The same cells (1 3 106 in 100 ml PBS) were injected into tail veins of WT and Cav1KO mice (strain B6.Cg-CAV1tm1mls/J) in an experimental metastasis assay. Successful injection was verified by immediate bioluminescent imaging. In the second orthotopic xenograft experiment (Figure 7D), LM-4175 cells prepared as before were injected into mammary glands of lethally irradiated WT and Cav1KO mice (strain STOCK Cav1tm1MIs/J), After 7 days, tumors and surrounding stroma were extracted and subcutaneously transplanted to nude mice. Alternatively, tumor architecture was analyzed using SHG imaging. In the subcutaneous tumorigenicity assay, 106 LM-4175 tumor cells were unmixed or mixed 1:1 with Cav1WT or Cav1KO pMEFs, or with Cav1KO pMEFs infected with p190RhoGAPshRNA lentiviral vector (+p190shKO MEFs). Cells were resuspended in a 1:1 mix of PBS and Matrigel (200 ml), and injected subcutaneously into anaesthetized nude mice using a 25-gauge needle. For bioluminescent imaging, mice were injected with luciferin (17.5 mg/ml), and after 20 min were placed in the IVIS Imaging System and ventral views captured. Tumor growth and metastasis formation were monitored at regular intervals. To facilitate metastasis detection in axillary/brachial lymph nodes, front limbs were secured with tape and the lower portion of the animal was shielded to block bioluminescence from the primary tumors. Exposure time for photon flux quantification was 0.2 s, but ranged from 0.2 s to 2min for metastasis detection. At the end of the *in vivo* analysis, luciferin-injected mice were killed and the organs extracted and reimaged *ex vivo* to detect metastatic foci. Images acquired from multiple exposure times were used to manually quantify every visible metastatic focus. Small metastatic foci could be detected by adjusting the scale of photon flux in Living Image 3.2 software. Extracted primary tumors were frozen in tissue-Tec and prepared for histology.

Alternatively, LM-4175, alone or mixed with pMEFs, was subcutaneously injected. Tumor growth and metastasis were assessed by bioluminescence with the IVIS Imaging System. Briefly, mice were injected with luciferin (17.5 mg/ml) and after 20 min were placed in the IVIS Imaging System and ventral views captured. Tumor growth and metastasis formation were monitored at regular intervals. Exposure time for photon flux quantification was 0.2 s but ranged from 0.2 s to 2 min for metastasis detection. At the end of the *in vivo* analysis, luciferin-injected mice were killed and the organs extracted and reimaged *ex vivo* to detect metastatic foci. Images acquired from multiple exposure times were used to manually quantify every visible metastatic focus. Small metastatic foci could be detected by adjusting the scale of photon flux in Living Image 3.2. Extracted primary tumors were frozen in tissue-Tec and prepared for histology. Alternatively, tumor architecture was analyzed by SHG imaging.

## Image Analysis of Immunofluorescence-Labeled Cells and Matrices

Images were captured with Leica confocal microscopes. EF was determined with MetaMorph (Universal Imaging Corp).

## RNA Interference

RNA Interference-Mediated Knockdown of p190RhoGAP and Cav1 Sequences targeting mouse p190RhoGAP were cloned into short hairpin RNA (shRNA) vector pSuper.Retro.Neo+GFP (Oligoengine). The sequences, obtained from GenBank/EMBL/DDBJ under accession number NM_172739, were as follows: sequence#1 (SEQ ID NO: 3), nucleotides 2935-2953, 5'-GTTATGGACGCAACATTAA-3'; sequence#2 (SEQ ID NO: 4), nucleotides 1225-1243, 5'-ACAGGAACTTCGATGATCA-3'; and sequence#3 (SEQ ID NO: 5), nucleotides 1955-1963, 5'-GACACCAACCTTCCAACCC-3'. The nontargeting control (scrambled) sequence was SEQ ID NO: 6: 5'- GCGCGCTTTGTAGGATTCG-3'. Retroviral supernatants were generated by transfecting 293T/17 cells with each shRNA and pSVc2 vector (White et al. J Virology, 1999, 73 (4) 2832-2840) using the Fugene 6 transfection reagent (Roche). Cav1KO MEFs were infected with retroviral supernatants and high GFP-expressing cells were sorted (approx. 15% of the cell population). To silence p190RhoGAP in primary Cav1KO MEFs for the subcutaneous tumorigenicity assay, sequence#1 (SEQ ID NO: 3) was cloned into pLVX-shRNA2, which contains a ZsGreen1 reporter (Clontech), and primary KO MEFs were infected with supernatants derived from HEK293T cells. Infection efficiency was monitored by ZsGreen1 expression. A representative immunoblot confirming silencing efficiency is presented in Figure 7 Ca. An alternative Cav1 siRNA was designed (sequence #2 (SEQ ID NO: 2), Fig.12B: 5'-GACGTGGTCAAGATTGACTTT-3') corresponding to bases 254-277 of the human Cav1. This sequence was also cloned into pLVX-shRNA2. Normal and tumor-associated fibroblasts were infected with supernatants derived from HEK293T cells. Infection efficiency was monitored by ZsGreen1 expression.

**Liquid Chromatography and Tandem Mass Spectrometry**

<u>ITRAQ (Isobaric tag for relative and absolute quantitation) Labeling, Identification, and Quantification of ECM Proteins by Liquid Chromatography Coupled to Tandem Mass Spectrometry</u>

FDMs from WT and KO MEFS were extracted as described above (Method #A). Alternatively, FDMs were extracted with three successive treatments with hypotonic PBS (diluted 1:5) containing 0.5% w/v sodium deoxycholate (DOC) (Method #B). Extracted FDMs were extensively washed with PBS, scraped off the plate, and proteolyzed with 4 mg trypsin (30 mM Tris, pH8) overnight at 37°C. The resulting soluble protein extracts were quantified by Bradford assay. Each sample was then denatured and digested as described in the iTRAQ reagents protocol. For protein digestion, modified porcine trypsin (Promega) was added at a final trypsin: protein ratio of 1:50. After digestion overnight at 37°C, samples were labeled with iTRAQ tags as follows: proteins obtained by "Method A" were labeled with WT-iTRAQ 115 and KO-iTRAQ 114; proteins prepared by "Method B" were labeled with WT-iTRAQ 116 and KO-iTRAQ 117. After labeling, the samples were vacuum dried and dissolved in buffer A (0.5% acetic acid) for desalting and removal of excess iTRAQ reagent in reverse-phase (RP) C-18 cartridges. The resulting tryptic peptide mixtures were separated by nano-liquid chromatography coupled to mass spectrometry for protein identification. Peptides were injected onto a C-18 RP nano-column (100 mm I.D. and 12 cm; Mediterranea sea, Teknokroma) and analyzed on a continuous acetonitrile gradient consisting of 0%-43% B for 140 min, 50%-90% B for 1 min (B = 95% acetonitrile, 0.5% acetic acid). Peptides were eluted from the RP nano-column at a flow rate of 300 nl/min into an emitter nanospray needle for real-time ionization and peptide fragmentation on an LTQ-Orbitrap mass spectrometer (Thermo Fisher, San José, CA, USA). An enhanced FT-resolution spectrum (resolution = 60000) followed by the MS/MS spectra of the three most intense parent ions were analyzed during the chromatographic run (180 min). Each parent ion was fragmented by two dissociation methods: CID for peptide sequence analysis and HCD for quantification of the iTRAQ reporter low mass signals. Dynamic exclusion was set at 0.5 min. To identify proteins, tandem mass spectra were extracted and the charge state deconvoluted with Proteome Discoverer 1.0 (Thermo Fisher). All MS/MS samples were analyzed with SEQUEST (Thermo Scientific, version 1.0.43.2), MASCOT (MatrixScience, version 2.2.01), and X! Tandem (The GPM, thegpm.org; version 2007.01.01.1). Two mixed cleavages were allowed, and an error of 15 ppm or 0.8 Da was set for full MS and MS/MS spectra searches, respectively. Phosphorylation on Ser, Thr, or Tyr was specified as a variable modification. Scaffold (version _3_00_03, Proteome Software Inc., Portland, OR, USA) was used to validate MS/MS peptide and protein identifications. Protein probabilities were assigned by the Protein Prophet algorithm. Proteins that contained similar peptides and could not be differentiated based on MS/MS analysis alone were grouped to satisfy the principles of parsimony.

**Microscopy Techniques**

For 3D timelapse video microscopy, cells were seeded in FDMs and imaged for 12 hr. Centroids were tracked with MetaMorph.

<u>Nanomechanical and Imaging Measurements by Atomic Force Microscopy</u>

Experiments were performed with a multimode atomic force microscope fitted with a Nanoscope V controller (Veeco Digital Instruments). Imaging and force spectroscopy were carried out in PBS (0.01 M, pH = 7.4). Images and force curves were obtained using cantilevers with a force constant kz0.01 N/m (Olympus BL-RC150VB-C1). The force constant was calibrated by the thermal tuning method (Butt and Jascke, 1995 Nanotechnology 6, 1-7). The sensitivity of the photodiode was calibrated from a deflection versus distance curve performed on a mica substrate (36.14 nm/V). Prior to measurements, the ECM was rinsed with deionized water and dried in gaseous $N_2$. Once the sample was mounted into the fluid cell of the microscope, 100 ml PBS was introduced into the liquid cell and the experiment proceeded. To acquire the force curves, we first recorded a 30 mm x 30 mm image of a representative area of the sample. We then selected points on the fiber force spectroscopy measurements. The force curves were acquired by approaching and retracting the tip toward the sample by 2 mm at a rate of 1 Hz. Each curve has 512 x 512 points. To prevent damage to fibers, tip excursion was stopped when the force reached 2 nN. The Young modulus was derived by the Hertz model,

$$F = \frac{4}{3} E_{eff} \sqrt{R} \delta^{3/2} \qquad (1)$$

where F is the applied load, R the tip radius (20 nm), E the effective Young modulus of the tip-sample interface, n the Poisson ratio (0.5 for soft materials), and d the indentation. The ECM stiffness is several orders of magnitude smaller than that of the AFM probe, so that $E \approx E_{ECM}$. Equation 1 requires transformation of the cantilever deflection dependence on sample displacement into force versus indentation curves. This is accomplished by determining the indentation from

$$\delta = \left(S_p - S_0\right) - \left(z - z_0\right) \qquad \textbf{(2)}$$

where Sp is the piezo movement, So the contact point, z the deflection recorded by the photodiode and $z_0$ the deflection offset. The measurements were performed at the point of maximum indentation, which in turn was limited by the maximum force applied.

FRAP

MEFs transfected with vinculin-GFP were plated overnight on the assorted FDMs. Two prebleach events were acquired before bleaching by stimulation with the SP5 scanner at 488 nm. Fluorescence recovery was monitored at 4 s intervals until the intensity reached a plateau. Fluorescence during recovery was normalized to the prebleach intensity. Relative recovery of vinculin-GFP at 3D-matrix adhesions and FAs was evaluated by comparing the half-times of fluorescence recovery toward the asymptote. Mobile and immobile fractions were calculated from comparison of intensity ratios in the bleached area before bleaching and after recovery. Graphs are representative of a minimum of three independent experiments in which between 6 and 15 adhesion structures were bleached.

Multiphoton Microscopy and Second Harmonic Generation

For MPE and SHG imaging of fixed, intact (nonsectioned), nonstained mammary glands and mammary tumors, we used an optical workstation built around a Nikon Eclipse TE300 (Provenzano et al., 2006 BMC Med. 4, 38). A Ti: sapphire laser excitation source (Spectra-Physics- Millennium/Tsunami, Mountain View, CA, USA), producing around 100 fs pulse widths and tuned to 890-900 nm, was used to generate both multiphoton excitation (cellular autofluorescence from FAD) and SHG. The beam was focused onto the sample with either a Nikon 40 3 Plan Fluor oil-immersion lens (N.A. = 1.4) or a Nikon 60 3 Plan Apo water-immersion lens (N.A. = 1.2). All SHG imaging was detected from the back-scattered SHG signal. Additionally, due to the fundamental differences between MPE (autofluorescence) and SHG signals, filtering can separate the emission signals. Using a 470 nm (cut-on) long pass (LP) filter, autofluorescence was discriminated from the total emission, while a 445nmnarrow band pass filter ($\pm$20 nm) was used to separate SHG(filters from TFI Technologies, Greenfield, MA, USA). For mammary tumors, a 520/535nm filter was used to detect GFP signals. All power settings were held constant during acquisition to allow comparison of intensities. Interestingly, some collagen fibers were visible using the LP filter. Acquisition was performed with WiscScan, a software acquisition package developed at LOCI (http://www.loci.wisc.edu/software/wiscscan). Image analysis for combined MPE-SHG was performed with ImageJ software, which was also used to quantify differences between SHG signals. Fluorescence intensity of collagen SHG in mammary tumors was measured in 1,000 mm$^2$ regions of interest drawn in the stroma adjacent to tumor cell islands; multiple regions were examined for each tumor. The angle of collagen fibers to the tumor boundary was determined for regions in which such a boundary was evident. Alignment was analyzed using Curvelet-Based Alignment Analysis software (www.loci.wisc.edu/software/curvelet-based-alignment-analysis), in which a line was drawn at the tumor/stroma boundary and collagen angles determined relative to that line. Quantitative data were graphed and statistically analyzed with GraphPad software.

Immunohistochemistry

Paraffin-embedded sections were deparaffinized in xylene, rehydrated through a graded series of ethanol and water and boiled in 10 mM sodium citrate-buffer for antigen retrieval. Sections were incubated with assorted antibodies overnight at 4°C. HRP secondary antibodies were detected with the Liquid DAB Substrate Pack (Biogenex, San Ramon, CA, USA) according to the manufacturer's instructions. Sections were counterstained with Harris modified hematoxylin (Thermo Fisher Scientific). Negative controls included omission of primary antibody and its substitution with normal rabbit or mouse IgG (Sigma-Aldrich). Collagen deposition and organization was visualized by standard Masson's trichrome staining or picrosirius red staining. To highlight fibrous structures, picrosirius red labeled samples were visualized with a Leica DM 2500 microscope fitted with an orthogonally oriented polarizer.

<u>Immunofluorescence and Confocal Microscopy of Melanoma Tissues</u>

Thin sections (4 mm) of cryopreserved tissue were first blocked for 10 min with 1% human immunoglobulins and then incubated for 1 hr with a mixture of primary antibodies from different species (for example, rabbit polyclonal antiserum against Cav-1, and HMB-45 monoclonal antibodies, or isotype-matched control antibodies). Primary antibodies were used at 1-5 mg/ml, followed by incubation with Cy5-labeled antimouse and Cy3-labeled antirabbit secondary antibodies. After blocking with 10% mouse immunoglobulins, samples were incubated with FITC-labeled anti-CD90 or anti-CD45, and with DAPI to visualize nuclei. Samples were imaged using an AOBS/SP2 inverted scanning confocal microscope (Leica Microsystems) fitted with a 633 PL-APO NA 1.3 immersion objective. Image processing and colocalization analyses were assessed with LCS-15.37 Leica Confocal Software.

**Tissue Samples, Cell Isolation, and Tumor Microarrays**

Primary fibroblasts from paired normal human kidney and tumor samples and breast tumor microarrays (TMAs) were obtained from Fox Chase Cancer Center's Biosample Repository (Philadephia, PA, USA). Samples were blindly scored by a certified pathologist. Colon carcinoma and normal colon tissues were from the Centro Nacional de Investigaciones Oncológicas, Madrid, Spain. Human melanoma tissue was taken with informed consent from patients undergoing surgery at the Hospital Gregorio Marañón, Madrid.

<u>Isolation of Primary Fibroblasts Associated with Normal and Tumor Breast and Kidney Samples</u>

To protect patient privacy, samples were decoded according to approved IRB procedures, whereas relevant clinical information was made available to the researchers upon request. Fresh surgical specimens (paired normal kidney and renal tumor tissue or nonpatient matched normal and tumor breast tissues) were minced and dissociated by incubation overnight at 37°C in 0.2% collagenase with agitation at 200 rpm. Fibroblasts were isolated by 10 min centrifugation at 200 g. Pellets were resuspended and serially filtered through 500 mm nylon mesh and 100 mm and 40 mm cell strainers. Isolated fibroblasts were cultured in high-glucose Dulbecco's modified Eagle medium (DMEM; Mediatech, Inc.) supplemented with 15% fetal bovine serum (FBS; Hyclone), 100 units/ml penicillin, and 100 mg/ml streptomycin (Mediatech, Inc.). Non-adherent material was removed after 8 hr. After characterization, kidney fibroblasts were immortalized using a combination of hTERT and bmi-1, while breast fibroblasts were used as primary fibroblasts. Both immortalized (at early passages) and primary cells were forced to produce their own matrix and subsequently grown in this 3D matrix (as opposed to a 2D plastic substrate) to allow them to reproduce their *in vivo* features.

**Membrane Fractionation**

Detergent-resistant and PM fractions were purified by standard procedures.

<u>Plasma Membrane Purification</u>

Briefly, cells were washed twice with buffer A (250 mM sucrose, 1 mM EDTA, 20 mM Tricine, pH 7.8) and collected and harvested in 3 ml buffer A. After centrifugation, cells were resuspended in 1 ml of buffer A and hand homogenized with 20 strokes. Homogenates were centrifuged at 1000 g for 10 min. The postnuclear supernatant (PNS) was layered (4 mg protein) on top of 23 ml of 30% Percoll in buffer A, and centrifuged at 84,000 g for 30 min. The plasma membrane fraction appeared as a visible band approximately 5.7 cm from the bottom of the tube. The membrane fraction was collected, diluted in buffer A and centrifuged at 105,000 g for 1 hr to remove Percoll. Purified plasma membranes in the resulting pellets were analyzed by immunoblot.

<u>Purification of Detergent-Resistant Membrane Fractions</u>

Briefly, MEFs were scraped off the plate and resuspended at 4°C in 2 ml Mes buffered saline (MBS) (25 mM MES, pH 6.5, 0.15 M NaCl, 1 mM PMSF plus 1% Triton X-100). Cells were homogenized on ice by a minimum of 10 strokes through a syringe (0.5 3 16 mm). The homogenate was adjusted to 40% sucrose by the addition of 2ml 80% sucrose in MBS (4 ml in total), and was transferred to a Beckman SW40 13 ml Ultraclear tube. 30% and 5% sucrose in MBS (4 ml each) were successively overlaid to form a 40-30%-5% discontinuous sucrose gradient. Homogenates were separated by centrifugation at 200,000 g for 18 hr a SW40 rotor (Beckman) at 4°C. Most Cav1 was contained in a light, scattered band confined to the 30%-5% sucrose interface, which excluded most cell proteins. Twelve 1 ml fractions were collected from the bottom of the tube. Proteins in each fraction were precipitated by addition of 1 ml of cold acetone and incubation overnight at 4°C. Samples were centrifuged at 16,000 g in a microcentrifuge, and the protein pellets were dried for 2 hr to eliminate all traces of acetone. Precipitated proteins were analyzed by SDS-PAGE and immunoblot.

Immunoprecipitation of p190RhoGAP

MEF plasma membranes were resuspended in lysis buffer (5mM Tris-HCl pH 7.5, 100 mM NaCl, 0.5% sodium deoxycholate, 0.1% SDS, 1% NP-40 containing 0.5mMPMSF, 1mMNa3VO4, 1 mg/ml aprotinin, 1 mg/ml leupeptin, and 10mMNaF) and incubated overnight with 4 mg anti-p190RhoGAP antibody. Fifty microliter protein G-agarose beads were added and samples incubated for 3 hr at 4°C. After washing and boiling in sample buffer, eluted proteins were analyzed by 7.5% SDS-PAGE and immunoblot.

**Statistics**

Error bars depict standard error of the mean (SEM). Statistical significance was determined with GraphPad Prism by unpaired Student's t test or Mann- Whitney test as indicated; * $p < 0.05$, ** $p < 0.01$, *** $p < 0.001$.

**Claims**

1. Use of *caveolin-1* in tumor-associated fibroblasts as biomarker of tumor progression.

2. Use according to claim 1 comprising at least one further biomarker selected from:

   smooth muscle actin, CD90 and vimentin.

3. Use according to claim 2 wherein the biomarker is smooth muscle actin.

4. Method for the diagnosis and/or prognosis of tumor progression comprising the following steps:

   a) detection and/or quantification of an expression product of *caveolin-1* gene in tumor-associated fibroblasts of a sample isolated from a subject,
   b) comparison of the expression levels of *caveolin-1* detected and/or quantified in step (a) with standard values,
   c) finding a significant overexpression of *caveolin-1* in the comparison of step (b), and
   d) attributing the significant overexpression of *caveolin-1* found in step (c) to a poor prognosis.

5. Method according to claim 4 wherein step (a) further comprises the detection and/or quantification of the expression product of at least one of the following biomarkers:

   smooth muscle actin, CD90 and vimentin.

6. Method according to claim 5 wherein step (a) comprises the detection and/or quantification of the expression product of smooth muscle actin.

7. Method according to any of claims 4 to 6 wherein the expression product is an mRNA or a protein.

8. Method according to any of claims 4 to 7 wherein the tumor is an epithelial tumor, preferably of breast, kidney, colon or melanoma.

9. A kit consisting essentially of specific primers and/or probes and/or antibodies for detecting and/or quantifying *caveolin-1* expression; and specific primers and/or probes and/or antibodies for detecting and/or quantifying the expression of at least one of the following genes: smooth muscle actin, CD90 and vimentin, preferably of smooth muscle actin.

10. A Caveolin-1 inhibitor for use as a medicament.

11. A Caveolin-1 inhibitor for use in the prevention of tumor progression.

12. The Caveolin-1 inhibitor according to claim 11, wherein the tumor is epithelial, preferably of breast, kidney, colon or melanoma.

13. The Caveolin-1 inhibitor according to any of claims 10 to 12, wherein said Caveolin-1 inhibitor is an interfering RNA or a blocking antibody.

14. The Caveolin-1 inhibitor according to claim 13 wherein said Caveolin-1 inhibitor is a small interfering RNA.

15. The Caveolin-1 inhibitor according to any of claims 13 or 14 wherein said Caveolin-1 inhibitor is SEQ ID NO: 1 or SEQ ID NO: 2.

FIG. 1A

FIG. 1B

FIG. 1 C

FIG. 1 D

Collagen-I gels

FIG. 1 Ea

Cav1WT MEFs    Cav1KO MEFs

FIG. 1 Eb

FIG. 1 Ec

FIG. 1 F

FIG. 1 G

FIG. 2

**FIG. 2 CONT.**

FIG. 2 CONT.

**a**

Cav1WT MEFs | Cav1KO MEFs

WT FDMs | KO FDMs | WT FDMs | KO FDMs

Vinculin-GFP

PB B 4 8 12 20 40 60 80 100

PB B 4 8 12 20 40 60 80 100

PB B 4 8 12 20 40 60 80 100

PB B 4 8 12 20 40 60 80 100

Time (Sec)

**b**

WT FDMs | Cav1WT MEFs
KO FDMs |
WT FDMs | Cav1KO MEFs
KO FDMs |

Normalized intensity

Vinculin-GFP

Cav1WT MEFs

**c**

Vinculin-GFP

Mobile Fraction

** | *

WT FDMs | KO FDMs | WT FDMs | KO FDMs

Cav1WT MEFs | Cav1KO MEFs

**FIG. 3**

**FIG. 3**

C    Fibronectin 3D matrices     Thresholded FN fibers

D

FDMs from Cav1KO MEFs

+Cav1WT

+Cav1Y14F

50 µm

35,57%

22,14%

Fibers (%)

Orientation angle

EP 2 544 003 A1

FIG. 3 E

FIG. 3 F

FIG. 3 G

**a**

| | SHG | Autofluorescence | Composite |

Cav1WT

Cav1KO

Mammary acini vicinity

**b**

| | PR | PR (polarized light) |

**c**

SHG signal intensity

Mammary acini vicinity

**FIG. 4 A**

FIG. 4 CONT.

**B**

Fibronectin Actin Nuclei

Cav1KO MEFs
p190RhoGAP shRNA

Scramble shRNA

**C**

Scramble shRNA

p190 RhoGAP shRNA

Rac-GTP

Total Rac

Cav1KO MEFs

**D**

Cav1KO MEFs
—⊗— Scramble shRNA
—▲— p190RhoGAP shRNA

Contraction index (%) vs Hours

*  **  #

**E**

Fibronectin

Cav1KO MEFS
p190 RhoGAP shRNA

Scramble shRNA

50 µm

FIG. 4 CONT.

FIG. 4 G CONT.

FIG. 4 CONT.

**H**

| | Cav1WT MEFs | Cav1KO MEFs | KO MEFs+empty | KO MEFs+Cav1 |

IP: p190RhoGAP
IB: 4G10 — 190 kDa

IP: p190RhoGAP
IB: p190RhoGAP — 190 kDa

Input
IB: p190RhoGAP — 190 kDa

Legend:
□ Cav1WT MEFs
■ Cav1KO MEFs
▥ KO MEFs+empty
▤ KO MEFs+Cav1

Amount of Phospho-p190RhoGAP (% change relative to control)

P-p190RhoGAP

**FIG. 4 I**

FIG. 4 I CONT.

FIG. 5

FIG. 5 CONT.

**E  KIDNEY CARCINOMA**

**F**

Figure panels E–H.

**FIG. 5 CONT.**

FIG. 6 A

FIG. 6 B

**FIG. 6 C**

FIG. 6 D

FIG. 7

FIG. 7 A d

FIG. 7 B a

Day 1     Day 2     Day 9     Day 102

Irradiation   WT/KO    WT/KO    WT/KO   Nude    Nude

LM-4175

Mammary gland xenografts  ⟶  Transplantation  ⟶  Tumor growth and metastasis formation

**FIG. 7 B b**

**FIG. 7 B c**

**FIG. 7 B d**

**FIG. 7 C a**

**FIG. 7 C b**

**FIG. 7 C c**

*in vivo*    *ex vivo*    *Intra tumoral*

LM-4175 only

+Cav1WT MEFs

+Cav1KO MEFs

+p190sh KOMEFs

50 µm

Fibronectin Hoechst     SMA

FIG. 7 C d

FIG. 8 A

FDMs

Fibronectin

**FIG. 8 B**

**FIG. 8 C**

FIG. 8 C

FIG. 8 E

FIG. 8 E CONT.

FIG. 9 A

FIG. 9 B

**FIG. 9 C**

|  | | Average protrusion number per cell |
|---|---|---|
| Cav1WT MEFs | ☐ WT FDMs | 2.72±0.07 ⎤ ** |
| | ■ KO FDMs | 3.32±0.04 ⎦ |
| Cav1KO MEFs | ▥ WT FDMs | 3.36±0.12 ⎤ *** |
| | ▤ KO FDMs | 4.75±0.04 ⎦ |

FIG. 9 D

Cav1WT MEFs

Cav1KO MEFs

FIG. 9 E

FIG. 10 A

**FIG. 10 B**

**FIG. 10 C a**

**FIG. 10 C b**

# iTRAQ™ Reagent design

Isobaric tag

| Reporter | Balance | Peptide Reactive Group |

**Extraction Method A:**

Cav1WT FDMs ➤ | 115 | 30 | PRG |

Cav1KO FDMs ➤ | 114 | 31 | PRG |

**Extraction Method B:**

Cav1WT FDMs ➤ | 116 | 29 | PRG |

Cav1KO FDMs ➤ | 117 | 28 | PRG |

**FIG. 10 D**

**FIG. 10 E**

Skin - 3 weeks

Cav1WT / Cav1KO

Tri. Masson / PR / PR (polarized light)

Mammary glands - Surrounding stroma

Cav1WT / Cav1KO

SHG / Autofluorescence / Composite

FIG. 11 A

FIG. 11 B

FIG. 11 C

FIG. 11 C

24h

FIG. 11 D a

**FIG. 11 D b**

Fibronectin (4 hours)

Rac Actin Hoechst

FIG. 11 E

FIG. 11 F

**FIG. 12 A**

**FIG. 12 B**

FIG. 12 C

#36  Hmb45 (white) Cav1

#97  Hmb45 (white) Cav1

FIG. 12 D

#20  Cav1    Cav1 Hmb45    Cav1 CD90

DIC    COL1 (white) Hoechst    FN (white) Hoechst

**FIG. 13 A**

**FIG. 13 B**

FIG. 13 C

FIG. 13 D

**FIG. 13 E**

Cav1WT MEFs      Cav1KO MEFs

**FIG. 14 A a**

FIG. 14 A b

FIG. 14 A c

FIG. 14 B a

**FIG. 14 B b**

**FIG. 14 B c**

**FIG. 14 C a**

**FIG. 14 C b**

**FIG. 14 D a**

**FIG. 14 D b**

Experimental metastasis assay

**FIG. 14 D c**

## EUROPEAN SEARCH REPORT

**Application Number**

EP 11 38 2228

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | JACKY G. GOETZ ET AL: "Biomechanical Remodeling of the Microenvironment by Stromal Caveolin-1 Favors Tumor Invasion and Metastasis", CELL, vol. 146, no. 1, 7 July 2011 (2011-07-07), pages 148-163, XP55014309, ISSN: 0092-8674, DOI: 10.1016/j.cell.2011.05.040 ----- | | INV. G01N33/574 C12N15/113 |
| X | US 2005/036986 A1 (THOMPSON TIMOTHY C [US]) 17 February 2005 (2005-02-17) | 10-13 | |
| Y | * abstract * * paragraphs [0017] - [0019], [0071] - [0074], [0077], [0090], [0097] - [0109] * ----- | 14,15 | |
| X | US 7 704 686 B2 (MORIMOTO CHIKAO [JP] ET AL) 27 April 2010 (2010-04-27) * abstract * * column 5, lines 1-22 * * column 12, lines 36-60 * ----- | 10,14 | |
| Y | SALAHALDIN TAHIR ET AL: "Caveolin-1 regulates VEGF-stimulated angiogenic activities in prostate cancer and endothelial cells.", CANCER BIOLOGY & THERAPY, vol. 8, no. 23, 1 December 2009 (2009-12-01), pages 2286-2296, XP55014706, ISSN: 1538-4047 * abstract * * page 87, column 1, paragraph 2 - column 2, paragraph 1 * ----- | 14,15 | |

**TECHNICAL FIELDS SEARCHED (IPC)**

G01N
C12N

-/--

The present search report has been drawn up for all claims

| Place of search | Date of completion of the search | Examiner |
|---|---|---|
| Munich | 14 December 2011 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 38 2228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SIMPKINS S A ET AL: "Contribution of Stroma to Breast Cancer Outcome", JOURNAL OF PATHOLOGY, JOHN WILEY & SONS LTD, GB, vol. 224, no. Suppl.1, 1 April 2011 (2011-04-01), page S8, XP009154721, ISSN: 0022-3417 * the whole document * | 9 | |
| A | KASPER M ET AL: "Immunoelectron microscopical characterization of the epithelioid type of smooth muscle cells in human glomus organs", ULTRASTRUCTURAL PATHOLOGY, HEMISPHERE, WASHINGTON, DC, US, vol. 21, no. 5, 1 September 1997 (1997-09-01), pages 425-430, XP009154724, ISSN: 0191-3123 * abstract * * table 1 * | 9 | |
| X | ISABELLE MERCIER ET AL: "Human breast cancer-associated fibroblasts (CAFs) show caveolin-1 down-regulation and RB tumor suppressor functional inactivation: Implications for the response to hormonal therapy", CANCER BIOLOGY & THERAPY, vol. 7, no. 8, 1 August 2008 (2008-08-01), pages 1212-1225, XP55014714, ISSN: 1538-4047, DOI: 10.4161/cbt.7.8.6220 | 1 | TECHNICAL FIELDS SEARCHED (IPC) |
| A | * the whole document * | 4-8 | |

-/--

The present search report has been drawn up for all claims

| Place of search | Date of completion of the search | Examiner |
|---|---|---|
| Munich | 14 December 2011 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 38 2228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | K. SAVAGE ET AL: "Caveolin 1 Is Overexpressed and Amplified in a Subset of Basal-like and Metaplastic Breast Carcinomas: A Morphologic, Ultrastructural, Immunohistochemical, and In situ Hybridization Analysis", CLINICAL CANCER RESEARCH, vol. 13, no. 1, 1 January 2007 (2007-01-01), pages 90-101, XP55014740, ISSN: 1078-0432, DOI: 10.1158/1078-0432.CCR-06-1371 * abstract * * page 92, column 2, paragraphs 4,5 * * page 94, column 2, paragraphs 1,2 * * page 99, paragraph bridging left and right hand columns * ----- | 1-15 | |
| A | HEHLGANS STEPHANIE ET AL: "Caveolin-1: an essential modulator of cancer cell radio-and chemoresistance.", AMERICAN JOURNAL OF CANCER RESEARCH 2011 LNKD- PUBMED:21984970, vol. 1, no. 4, 20 March 2011 (2011-03-20), pages 521-530, XP55014745, ISSN: 2156-6976 * the whole document * ----- | 1-15 | TECHNICAL FIELDS SEARCHED (IPC) |

The present search report has been drawn up for all claims

| Place of search | Date of completion of the search | Examiner |
|---|---|---|
| Munich | 14 December 2011 | Thumb, Werner |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 11 38 2228

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2011

| Patent document cited in search report | | Publication date | Patent family member(s) | | Publication date |
|---|---|---|---|---|---|
| US 2005036986 | A1 | 17-02-2005 | NONE | | |
| US 7704686 | B2 | 27-04-2010 | CA | 2551912 A1 | 14-07-2005 |
| | | | EP | 1709450 A2 | 11-10-2006 |
| | | | JP | 2007517491 A | 05-07-2007 |
| | | | US | 2007259824 A1 | 08-11-2007 |
| | | | WO | 2005063170 A2 | 14-07-2005 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 07915384 B

**Non-patent literature cited in the description**

- **YAMADA ; CUKIERMAN.** *Cell,* 2007, vol. 130, 601-610
- **ENGLER et al.** *Science,* 2009, vol. 324, 208-212
- **KRIEG et al.** *Nat. Cell Biol.,* 2008, vol. 10, 429-436
- **RONNOV-JESSEN ; BISSELL.** *Trends Mol. Med.,* 2008, vol. 15, 5-13
- **LEVENTAL et al.** *Cell,* 2009, vol. 139, 891-906
- **FRIEDL ; GILMOUR.** *Nat. Rev. Mol. Cell Biol.,* 2009, vol. 10, 445-457
- **PROVENZANO et al.** *Biophys. J.,* 2008, vol. 95, 5374-5384
- **GAGGIOLI et al.** *Nat. Cell Biol.,* 2007, vol. 9, 1392-1400
- **KARNOUB et al.** *Nature,* 2007, vol. 449, 557-563
- **ORIMO et al.** *Cell,* 2005, vol. 121, 335-348
- **PARTON ; SIMONS.** *Nat. Rev. Mol. Cell Biol.,* 2007, vol. 8, 185-194
- **GOETZ et al.** *J. Cell Biol.,* 2008, vol. 180, 1261-1275

- **GRANDE-GARCIA et al.** *J. Cell Biol.,* 2007, vol. 177, 683-694
- **GOETZ et al.** *Cancer Metastasis Rev.,* 2008, vol. 27, 715-735
- **SLOAN et al.** *Am. J. Pathol.,* 2009, vol. 174, 2035-2043
- **WITKIEWICZ et al.** *Am. J. Pathol.,* 2009, vol. 174, 2023-2034
- **DEWEVER O et al.** *Int J Cancer.,* 2008, vol. 123 (10), 2229-38
- **GOH, L.L. ; ED, M.** *PLoS ONE,* 2010, vol. 5 (8), e1409
- **WENNERBERG, K. et al.** *Current Biology,* 2003, vol. 13 (13), 1106-1115
- **CEREZO et al.** *Mol. Cell. Biol.,* 2009, vol. 29, 5046-5059
- **MINN et al.** *Nature,* 2005, vol. 436, 518-524
- **WHITE et al.** *J Virology,* 1999, vol. 73 (4), 2832-2840
- **BUTT ; JASCKE.** *Nanotechnology,* 1995, vol. 6, 1-7
- **PROVENZANO et al.** *BMC Med.,* 2006, vol. 4, 38

SEQUENCE LISTING

<110> CENTRO NACIONAL DE INVESTIGACIONES CARDIOVASCULARES

<120> CAVEOLIN-1 IN TUMOR-ASSOCIATED FIBROBLASTS AS BIOMARKER FOR TUMOR
      PROGRESSION

<130> EP1997.10

<160> 6

<170> PatentIn version 3.5

<210> 1
<211> 19
<212> DNA
<213> Mus musculus

<400> 1
aaccagaagg gacacacag                                                    19

<210> 2
<211> 21
<212> DNA
<213> Homo sapiens

<400> 2
gacgtggtca agattgactt t                                                 21

<210> 3
<211> 19
<212> DNA
<213> Mus musculus

<400> 3
gttatggacg caacattaa                                                    19

<210> 4
<211> 19
<212> DNA
<213> Mus musculus

<400> 4
acaggaactt cgatgatca                                                    19

<210> 5
<211> 19
<212> DNA
<213> Mus musculus

<400> 5
gacaccaacc ttccaaccc                                                    19

<210> 6
<211> 19
<212> DNA
<213> Artificial Sequence

<220>
<223> Scrambled siRNA sequence.

<400> 6
gcgcgctttg taggattcg                                                    19